# EUROPÄISCHE PATENTSCHRIFT

(11) **EP 0 782 995 B1**
(45) Veröffentlichungstag und Bekanntmachung des Hinweises auf die Patenterteilung: **16.08.2001**
(21) Anmeldenummer: 96120713.1
(22) Anmeldetag: 21.12.1996
(51) Int. Cl.: C07D 407/10, C07D 407/12, C09K 19/58, C09K 19/34

(54) **Optisch aktive Bis-Methyldioxan-Derivate und ihre Verwendung als elektrooptische Stoffe**
Optically active Bis-methyldioxan derivatives and their use for electro-optical displays
Dérivés de bis-méthyldioxane optiquement actifs et leur utilisation pour matériel électro-optique

(30) Priorität: 02.01.1996 CH 396
(43) Veröffentlichungstag der Anmeldung: 09.07.1997
(73) Patentinhaber: Rolic AG, 6301 Zug (CH)
(72) Erfinder: Buchecker, Richard, 8008 Zurich (CH); Fuenfschilling, Jürg, 4054 Basel (CH)
(74) Vertreter: Groner, Manfred

(56) Entgegenhaltungen:
- EP-A- 0 154 840
- EP-A- 0 182 054
- EP-A- 0 315 014
- EP-A- 0 457 105
- EP-A- 0 732 330
- WO-A-91/16321
- DE-A- 3 715 029
- DE-A- 4 444 701
- MOLECULAR CRYSTALS AND LIQUID CRYSTALS (INC. NONLINEAR OPTICS ), Bd. 204, 1.Juli 1991, Seiten 37-42, XP000236762 YU L J ET AL: "SYNTHESIS AND PROPERTIES OF THERMOTROPIC LIQUID CRYSTALS BIS (4-(5-ALKYL-1,3-DIOXAN-2-YL)PHENYL) TEREPHTHALATE"

## Beschreibung

Die vorliegende Erfindung betrifft neue flüssigkristalline, optisch aktive Bis-Methyldioxane, sowie flüssigkristalline Mischungen, welche solche Verbindungen enthalten, die Verwendung solcher Verbindungen und Mischungen für optische und elektro-optische Vorrichtungen, und elektro-optische Vorrichtungen enthaltend solche Verbindungen.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten Zellen, besonders die aktiv angesteuerten Zellen, z.B. TFT-Zellen ("thin film transistor") wichtig geworden. Neben den genannten Zelltypen, welche auf der Verwendung von nematischen oder cholesterischen Flüssigkristallen beruhen, sind in letzter Zeit Zelltypen, welche auf dem Prinzip von chiral getilteten smektischen Phasen beruhen, bekannt geworden. Dazu gehören, SSF-Zellen (surface stabilized ferroelectric), SBF-Zellen (short-pitch bistable ferroelectric) oder DHF-Zellen (deformed helix ferroelectric). Für diese Zelltypen werden als getiltete smektische Phasen im allgemeinen chirale smektische C (S_{C}*) Phasen bevorzugt, welche besonders grosse Ansprechgeschwindigkeiten ermöglichen.

Die Materialien für Anzeigevorrichtungen auf der Basis von chiral getilteten smektischen Phasen können vorzugsweise aus einem Material mit getilteter smektischer Phase, meist einer S_{C} Phase, und einem oder mehreren optisch aktiven Dotierstoffen bestehen. Die Verwendung solcher chiraler Dotierstoffe in ferroelektrischen Flüssigkristallen dient vor allem zur Erzeugung oder Veränderung einer Verdrillung der getilteten smektischen Phase und zur Induzierung einer spontanen Polarisation. Die chiralen Dotierstoffe sollten eine ausreichende thermische und photochemische Stabilität besitzen, mit dem getilteten smektischen Flüssigkristall gut kompatibel sein, eine möglichst niedrige Viskosität aufweisen und den Mesophasenbereich nicht zu stark einschränken. Ferner ist eine möglichst hohe spontane Polarisation und je nach Zellentyp ein eher kleines (SSF-Zellen) oder ein möglichst hohes Verdrillungsvermögen (SBF-, DHF-Zellen) erwünscht.

Als chirale Dotierstoffe kommen im Prinzip auch Verbindungen in Frage, welche selbst keine getiltete Phase oder überhaupt keine Mesophase aufweisen. Dies ist besonders häufig bei Dotierstoffen mit einem sehr hohen Verdrillungsvermögen der Fall. Solche Dotierstoffe haben jedoch meist den Nachteil, dass sie den getilteten Phasenbereich der Wirtsmischung, der sie zugefügt werden, stark verkleinern.

Es ist bekannt, Dioxan-Derivate für elektro-optische Verwendungen einzusetzen. So sind aus Molecular Crystals and Liquid Crystals, Bd 204, 1.7.1991, Seiten 37 - 42, Dioxan-Derivate bekannt, die gemäss den Angaben der Autoren zur Herstellung ferroelektrischer Flüssigkristalle geeignet sind. Diese Vorveröffentlichung offenbart jedoch nur achirale Verbindungen ohne ferroelektrischen Flüssigkristalleigenschaften. Darüber hinaus gibt diese Publikation auch keinen Hinweis darauf, wie die offenbarten Verbindungen zur Herstellung chiraler Produkte mit ferroelektrischen Eigenschaften modifiziert werden können. Weitere Bis-Dioxan-Derivate für elektro-optische Anwendungen sind in DE-A 37 15 029 und EP-A 0 732 330 beschrieben, wobei die in der DE-A 37 15 029 offenbarten Verbindungen je einen endständigen Cyano-Substituenten und die aus der EP-A 0 732 330 bekannten Verbindungen je eine die beiden Dioxan-Ringe miteinander verbindende Alkylengruppe besitzen. Die in diesen beiden Vorveröffentlichungen offenbarten Verbindungen zeigen ebenfalls keine ferroelektrischen Eigenschaften, wobei die Verbindungen aus der EP-A 0 732 330 noch zusätzlich nur in einem engen Anwendungsbereich einsetzbar sind und wobei Mischungen enthaltend solche Verbindungen einen verhältnismässig niedrigen Klärpunkt besitzen.

Der Erfindung liegt nun die Aufgabe zu Grunde, neue Verbindungen bereitzustellen, welche ferroelektrische Eigenschaften besitzen oder solche in flüssigkristallinen Mischungen, die die Verbindungen enthalten, induzieren, und die vorgenannten Nachteile nicht oder nur in geringem Masse aufweisen.

Die erfindungsgemässen Verbindungen, welche sich durch ein hohes Verdrillungsvermögen und eine vergleichsweise hohe spontane Polarisation auszeichnen, weisen meist smektische Mesophasen, teilweise sogar Sc*-Phasen bei vergleichsweise hohen Temperaturen auf. Sie führen im Gemisch mit Sc-Basismischungen zu keiner oder nur zu einer relativ kleinen Absenkung der Sc*-Phase. Trotz ihrer Grundstruktur, bestehend aus fünf Ringen, sind sie in Sc-Grundmischungen überraschend gut löslich. Aufgrund dieser Eigenschaften sind sie als Dotierstoffe für die Anwendung in ferroelektrischen Zellen, insbesondere in DHF-Zellen, hervorragend geeignet. Daneben sind sie aber auch für cholesterische Mischungen, insbesondere bei Anwendungen als cholesterische Filter oder als Polarisatoren, einsetzbar.

Es sind dies Verbindungen der allgemeinen Formel 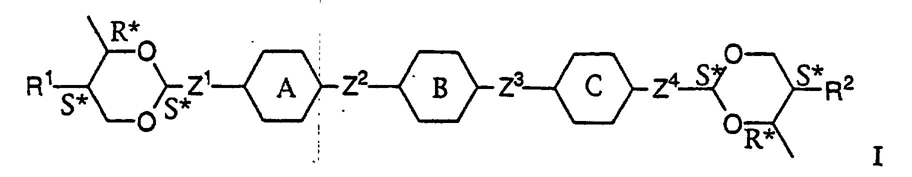 worin
- R¹ und R²: unabhängig von einander Alkyl oder Alkenyl, in welchen gegebenenfalls eine oder zwei nicht benachbarte Methylengruppen durch -O-, -COO-und/oder -OOC- ersetzt sind und/oder eines oder mehrere Wasserstoffatome durch Fluor, Chlor oder Cvano ersetzt sind. bedeuten: einer der Ringe
- A, B und C: ein gegebenenfalls mit 1 oder 2 Fluoratomen substituiertes 1,4-Phenylen, Pyridin-2,5-diyl oder Pyrimidin-2,5-diyl, und die beiden anderen Ringe unabhängig voneinander gegebenfalls mit 1 oder 2 Fluoratomen substituiertes 1,4-Phenylen oder trans-1,4-Cyclohexylen bedeuten;
- Z¹ und Z⁴: unabhängig voneinander eine Einfachbindung, -CH₂CH₂-, -CH₂O-, -OCH₂-, -C≡C-, -(CH₂)₄-, -(CH₂)₃O-, -O(CH₂)₃-, -CH=CH-CH₂O-, -OCH₂-CH=CH-, -CH=CH-(CH₂)₂- oder -(CH₂)₂CH=CH-, mit der Massgabe, dass das Sauerstoffatom nicht direkt mit dem chiralen Dioxanring verknüpft ist, bedeuten;
- Z² und Z³: unabhängig voneinander eine Einfachbindung, -CH₂-CH₂-, -O-CH₂-, -CH₂-O-, -COO-, -OOC-, -C≡C-, -(CH₂)₄-, -O(CH₂)₃-, -(CH₂)₃O-,-CH=CH-CH₂O-, -OCH₂-CH=CH-, -(CH₂)₂-CH=CH- oder -CH=CH-(CH₂)₂- bedeuten; und
- R* und S*: die relativen Konfigurationen der chiralen Kohlenstoffatome bedeuten.

Die in den Definitionen verwendeten Ausdrücke werden im folgenden erläutert:
Der Ausdruck "Alkyl oder Alkenyl, in welchen gegebenenfalls eine oder zwei nicht benachbarte Methylengruppen durch -O-, -COO- und/oder -OOC-ersetzt sind und/oder eines oder mehrere Wasserstoffatome durch Fluor, Chlor oder Cyano ersetzt sind" umfasst geradkettige und verzweigte (gegebenenfalls chirale) Reste wie Alkyl, 2-Alkenyl, 3-Alkenyl, 4-Alkenyl, Alkenyl mit endständiger Doppelbindung, Alkoxyalkyl, Alkenyloxyalkyl, Alkylcarbonyloxyalkyl, Alkoxycarbonylalkyl, Fluoralkyl, Difluoralkyl, Trifluoralkyl, Chloralkyl, Cyanoalkyl, 1-Methylalkyl, 2-Methylalkyl und dergleichen, jeweils mit höchstens 16 Kohlenstoffatomen, vorzugsweise höchstens 12 Kohlenstoffatome.
Beispiele bevorzugter Reste sind Methyl, Ethyl, Propyl, Butyl, Pentyl, Hexyl, Heptyl, Octyl, Nonyl, Decyl, Undecyl, Dodecyl, 1-Methylpropyl, 1-Methylheptyl, 2Z-Pentenyl, 2Z-Hexenyl, 2Z-Heptenyl, 2Z-Octenyl 3E-Pentenyl, 3E-Hexenyl, 3E-Heptenyl, 3E-Octenyl, 5E-Octenyl, 3-Butenyl, 4-Pentenyl, 5-Hexenyl, 6-Heptenyl, 7-Octenyl, Methoxyethyl, Methoxypropyl, Methoxybutyl, Ethoxyethyl, Propyloxyethyl, 2-Fluorpropyl, 2-Fluorpentyl, 2-Fluoroctyl, 2-Chlorhexyl, 2-Chloroctyl und dergleichen.
Der Ausdruck "gegebenenfalls mit 1 oder 2 Fluoratomen substituiertes 1,4-Phenylen" bedeutet 1,4-Phenylen, 2- bzw. 3-Fluor-1,4-phenylen, 2,3-Difluor-1,4-phenylen, 2,6- bzw. 3,5-Difluor-1,4-phenylen. Bevorzugte Fluor-substiuierte Ringe sind 2- bzw. 3-Fluor-1,4-phenylen oder 2,3-Difluor-1,4-phenylen.

Besonders bevorzugte Verbindungen der Formel I sind diejenigen, worin Z¹ und Z⁴ vorzugsweise eine Einfachbindung, -CH₂CH₂-, -CH₂O- oder -OCH₂-; und Z² und Z³ vorzugsweise eine Einfachbindung, -CH₂CH₂-, -CH₂O-, -OCH₂-, -COO- oder -OOC- bedeuten, wobei ein in den Gruppen Z¹ bis Z⁴ vorhandenes Sauerstoffatom vorzugsweise mit einem aromatischen Ring verknüpft ist. In ganz besonders bevorzugten Verbindungen sind höchstens zwei der Gruppen Z¹ bis Z⁴ verschieden von einer Einfachbindung.

Besonders bevorzugt sind Verbindungen der Formel I, worin die Ringe A, B und C unabhängig voneinander 1,4-Phenylen, 2- bzw. 3-Fluor-1,4-phenylen oder 2,3-Difluor-1,4-phenylen bedeuten; oder einer der Ringe A, B und C 1,4-Phenylen, 2- bzw. 3-Fluor-1,4-phenylen oder 2,3-Difluor-1,4-phenylen und die beiden anderen Ringe unabhängig voneinander 1,4-trans-Cyclohexylen, 1,4-Phenylen, 2- bzw. 3-Fluor-1,4-phenylen oder 2,3-Difluor-1,4-phenylen bedeuten.

Ganz besonders bevorzugte Verbindungen sind die Verbindungen der allgemeinen Formeln 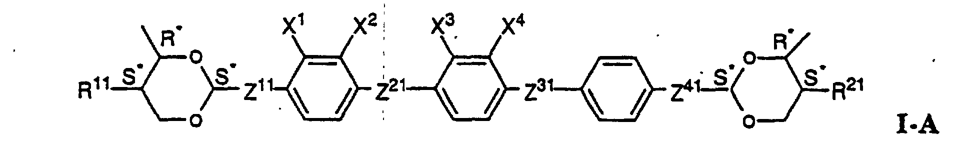 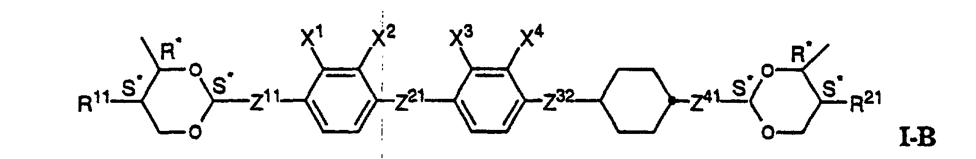 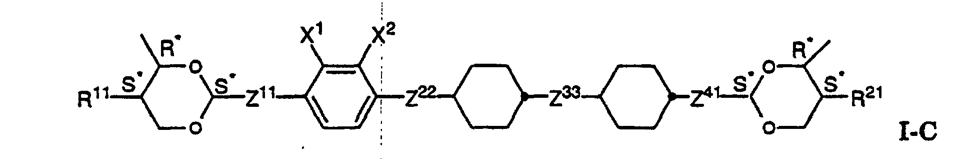 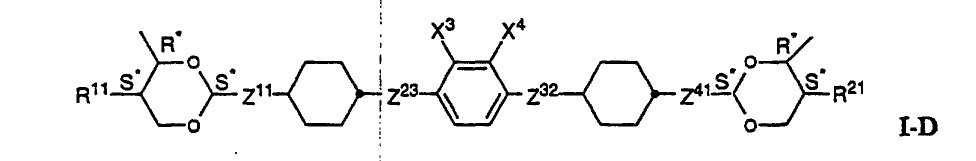 worin
- R¹¹ und R²¹: unabhängig voneinander Alkyl oder Alkenyl mit höchstens 12 Kohlenstoffatomen, in welchen gegebenenfalls eine Methylengruppe durch -O-, -COO-oder -OOC- ersetzt ist;
- Z¹¹, Z³³ und Z⁴¹: unabhängig voneinander eine Einfachbindung oder -CH₂CH₂-;
- Z²¹, Z³¹: eine Einfachbindung, -COO- oder -OOC-;
- Z²²,Z³²: unabhängig voneinander eine Einfachbindung, -CH₂CH₂- oder -OCH₂-;
- Z²³: eine Einfachbindung, -CH₂CH₂- oder -CH₂O-; und die Substituenten
- X¹ bis X⁴: unabhängig voneinander Wasserstoff oder Fluor bedeuten.

Die in Formel I und den folgenden Formeln mit (S*) und (R*) bezeichneten relativen Konfigurationen der chiralen Kohlenstoffatome bedeuten, dass die bezeichneten Kohlenstoffatome wie angegeben die (S)-oder (R)-Konfiguration aufweisen oder, dass alle bezeichneten Kohlenstoffatome die spiegelbildliche Konfiguration aufweisen. Die Formeln umfassen somit jeweils das Diastereoisomer, worin (S*) für die (S)-Konfiguration und (R*) für die (R)-Konfiguration steht, sowie dessen optischen Antipoden, worin (S*) für die (R)-Konfiguration und (R*) für die (S)-Konfiguration steht.

Insbesondere werden die Verbindungen der folgenden allgemeinen Formeln bevorzugt: 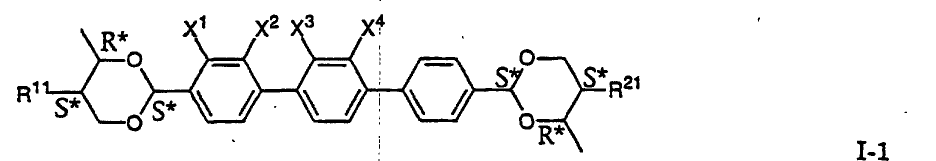 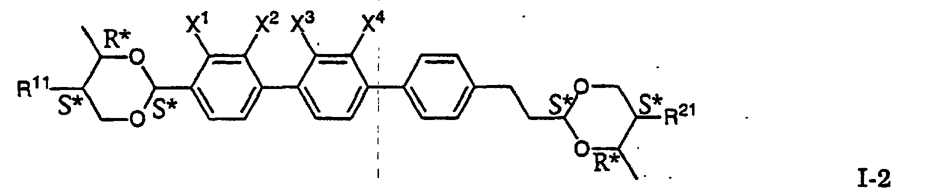 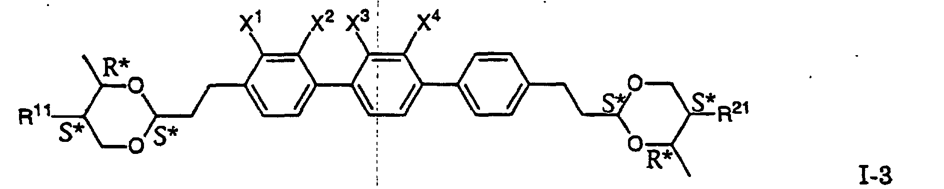 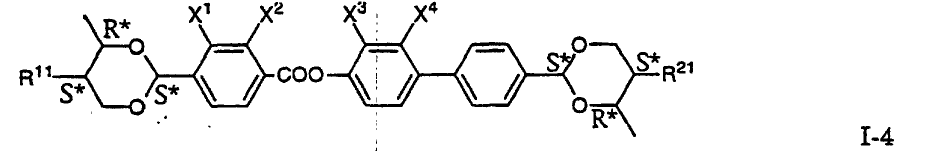 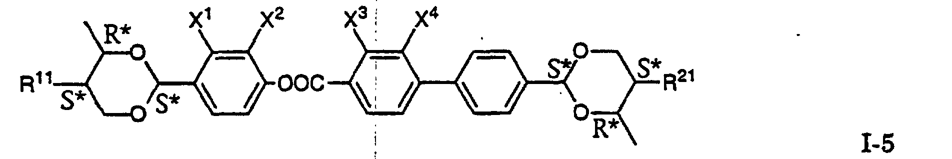 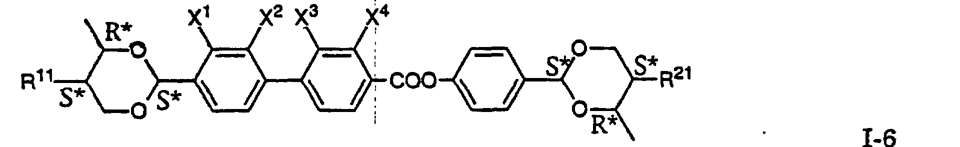 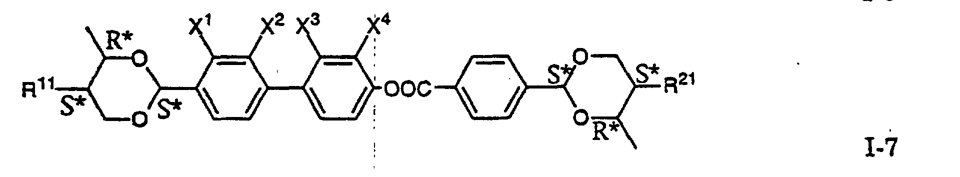 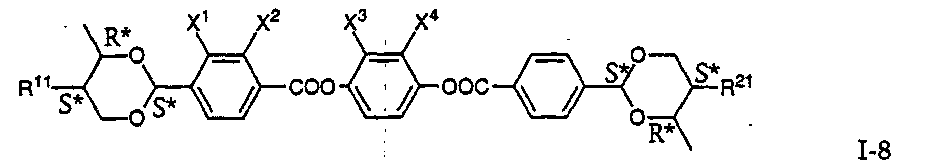 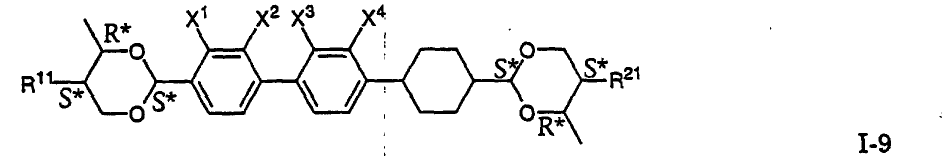 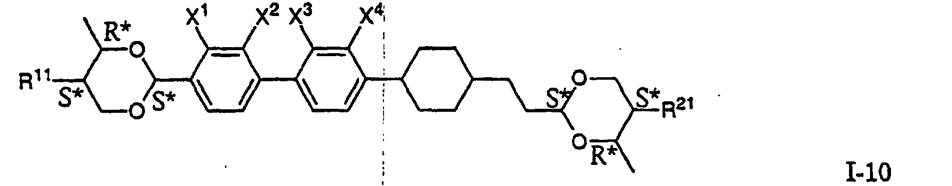 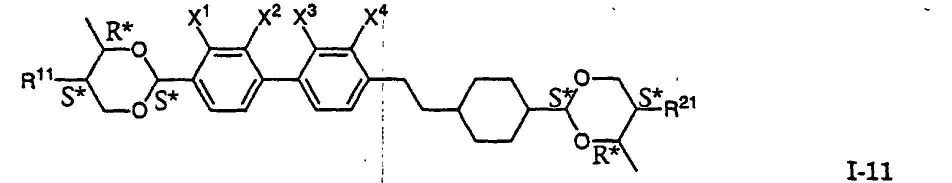 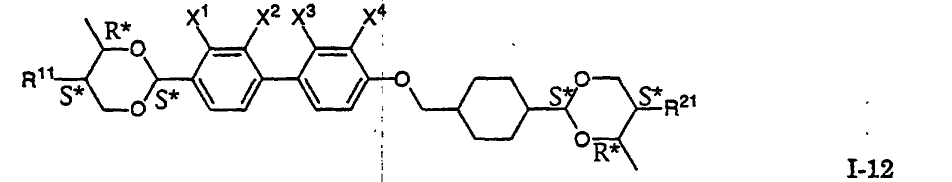 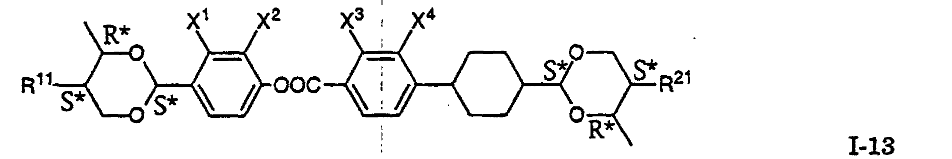 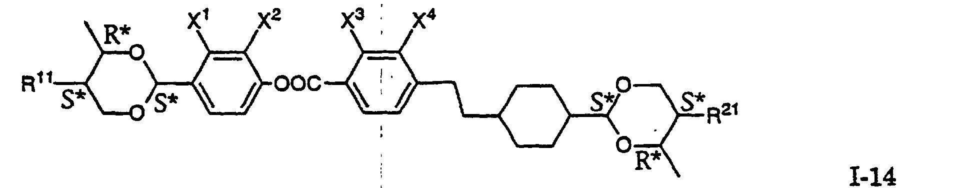 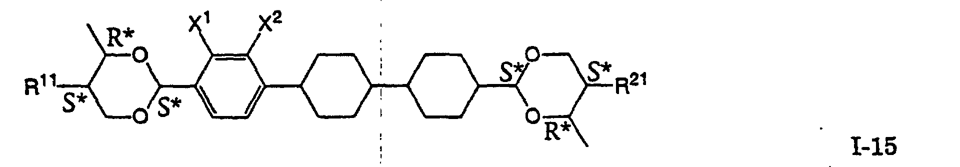 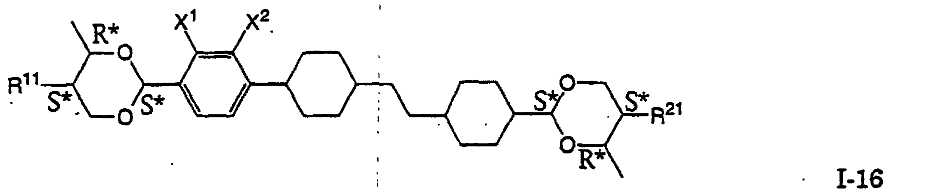 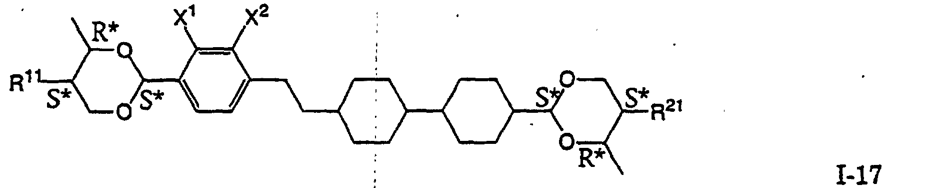 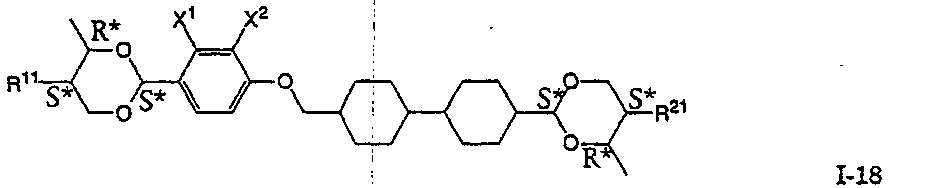 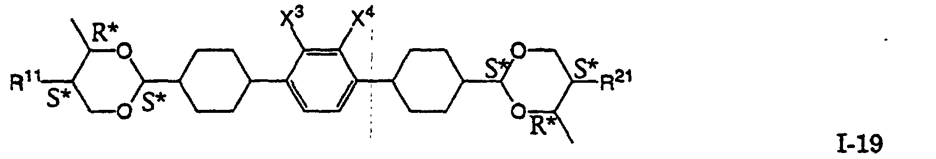 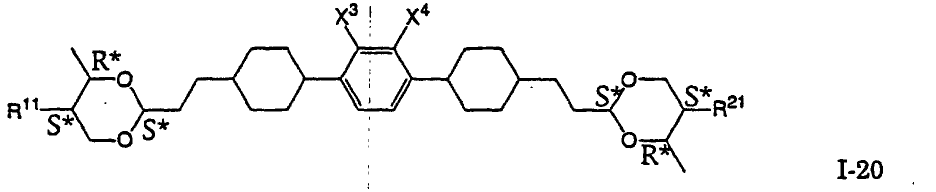 worin
- R¹¹ und R²¹: die oben angegebene Bedeutung haben; und die Substituenten
- X¹ bis X⁴: Wasserstoff oder Fluor bedeuten; vorzugsweise sind jeweils höchstens einer oder zwei der Substituenten X¹ bis X⁴ von Wasserstoff verschieden.

Die Verbindungen der allgemeinen Formel I können in an sich bekannter Weise, beispielsweise gemäss Schema 1, hergestellt werden. 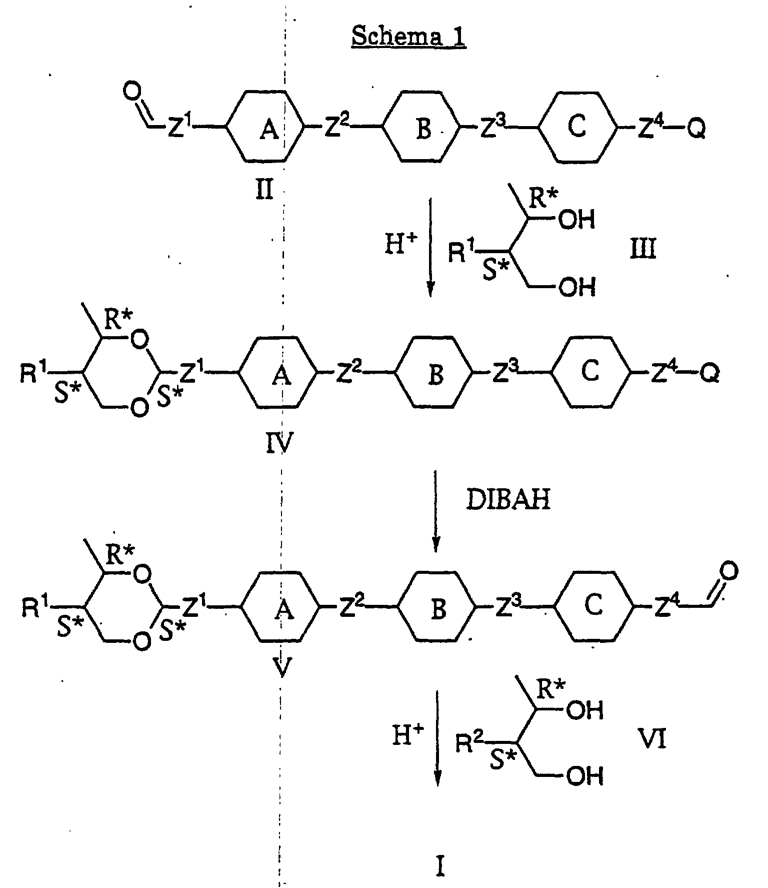 worin
- Q: -CN oder -COOR bedeutet;
- R: niederes Alkyl, wie Methyl, Ethyl, Propyl, i-Propyl, Butyl, 2-Butyl oder tert.-Butyl bedeutet;
und die übrigen Symbole obige Bedeutung haben.

Dabei wird ein Aldehyd der allgemeinen Formel II, welcher eine zweite, leicht in einen Aldehyd überführbare Gruppe Q enthält, zunächst mit einem Diol der allgemeinen Formel III zu einem Monoacetal der allgemeinen Formel IV umgesetzt. Danach wird durch Reduktion beispielsweise mit Diisobutylaluminiumhydrid (DIBAH) das Nitril oder der Ester der allgemeinen Formel IV in einen Aldehyd der Formel V übergeführt, der durch eine zweite Acetalisierung mit einem Diol der allgemeinen Formel VI das Diacetal der allgemeinen Formel I ergibt.

Wenn in R¹ und/oder R² Estergruppen vorhanden sind, wird vorzugsweise die Veresterung erst nach der Bildung der Dioxanringe erfolgen. Ferner kann, wenn R¹ und/oder R² Ethergruppen aufweisen, gewünschtenfalls auch die Veretherung nach der Bildung der Dioxanringe erfolgen.

Verbindungen der Formel I, worin Z² und/oder Z³ eine Estergruppe bedeuten, werden bevorzugt durch Veresterung von entsprechenden Teilstrukturen mit vorgeformten Dioxanringen hergestellt.

Des weiteren können diejenigen Verbindungen der allgemeinen Formel I, welche zwei benachbarte, direkt verknüpfte Phenylringe enthalten, durch Palladium katalysierte Kupplung beispielsweise eines geeignet substituierten aromatischen Halogenids oder Sulfonates der allgemeinen Formel VII mit einer geeignet substituierten Arylboronsäure der allgemeinen Formel VIII hergestellt werden (s. Schema 2). Solche Kupplungen sind mehrmals beschrieben worden, so zum Beispiel in Tetrahedron Letters 35, 3277 (1994) 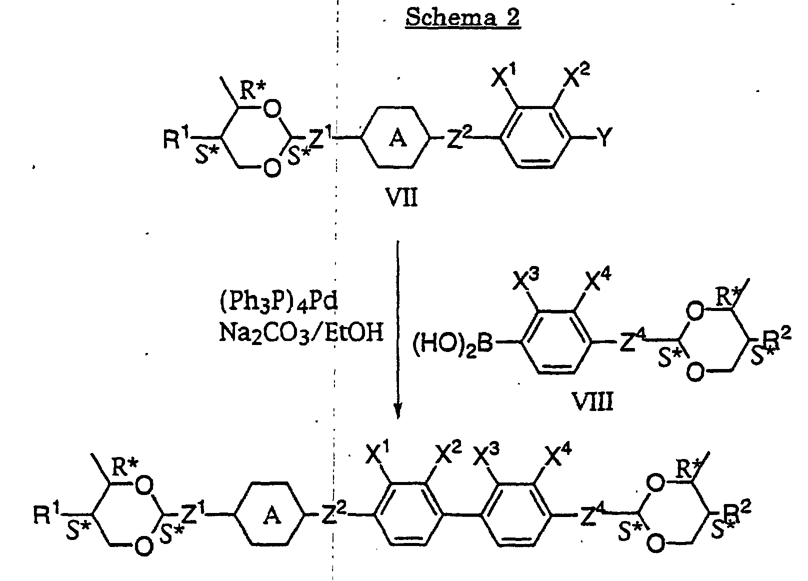 worin
- Y: Br, I oder -OSO₂CF₃ bedeutet; und
die übrigen Symbole obige Bedeutung haben.

Die zur Synthese der Verbindungen der Formel I benötigten Ausgangsmaterialien sind bekannt oder Analoga bekannter Verbindungen. So sind beispielsweise zahlreiche Nitrile oder Ester der Formel II als Zwischenstufen bei der Synthese von Flüssigkristallen beschrieben worden, beispielsweise in EP-A-0 122 389. Auch die Herstellung der Diole der Formel III ist aus der Literatur bekannt, beispielsweise aus Mol Cryst. Liq. Cryst *213*, 159 (1992)

Die Analoga der Ausgangsmaterialien der Formeln VII oder VIII, beispielsweise Verbindungen mit achiralem Dioxan- oder Dioxolanring, der üblicherweise als Schutzgruppe dient, sind ebenfalls aus der Literatur bekannt. Bekannt sind dem Fachmann Verknüpfungsreaktionen von Ringen durch Esterbildung, Etherbildung oder durch direkte aromatische Kupplungen via Boronsäuren, wie beispielsweise in Tetrahedron Letters, 35, 3277 (1994) beschrieben. Diese Reaktionen gehören zu den Standardmethoden bei der Synthese von Flüssigkristallen und sind daher dem Fachmann bestens vertraut.

Die erfindungsgemässen Verbindungen eignen sich als chirale Dotierstoffe für flüssigkristalline Gemische. Die Erfindung betrifft daher ebenfalls flüssigkristalline Gemische mit mindestens 2 Komponenten, welche dadurch gekennzeichnet sind, dass mindestens eine der Komponenten eine optisch aktive Verbindung der Formel I ist.

Vorzugsweise enthalten die erfindungsgemässen Gemische eine flüssigkristalline Einzelverbindung oder ein flüssigkristallines Gemisch als Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich aufweisen. Das flüssigkristalline Trägermaterial kann, wie erwähnt, eine Einzelverbindung oder ein Gemisch sein und besitzt vorzugsweise einen Klärpunkt von mindestens etwa 60°C. Besonders geeignet sind die Verbindungen der Formel I als chirale Dotierstoffe für Trägermaterialien mit nematischer, cholesterischer oder getilteter smektischer Phase, ganz besonders für Trägermaterialien mit einer achiralen oder chiralen smektisch C Phase. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, oder aus Landolt-Börnstein, Liquid Crystals, Bände IV 7a-d, viele davon sind zudem im Handel erhältlich.

Der Anteil an chiralem Dotierstoff der Formel I ist weitgehend durch das Verdrillungsvermögen, die spontane Polarisation und die gewünschte Ganghöhe bestimmt. Der Anteil an chiralem Dotierstoff kann daher je nach Anwendung in einem breiten Bereich variieren und beispielsweise etwa 0,1-40 Gew.-% betragen. Für Anzeigevorrichtungen auf der Basis von Flüssigkristallen mit getilteten smektischen Phasen ist im allgemeinen ein Anteil von etwa 1-30 Gew.-%, insbesondere von etwa 3-25 Gew.-%, an optisch aktiven Dotierstoffen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 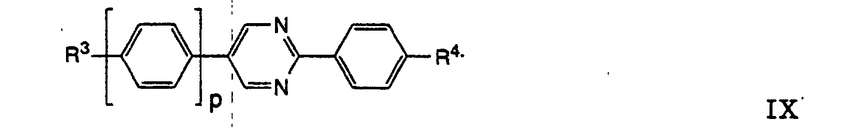 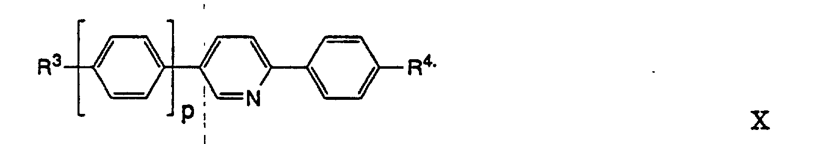 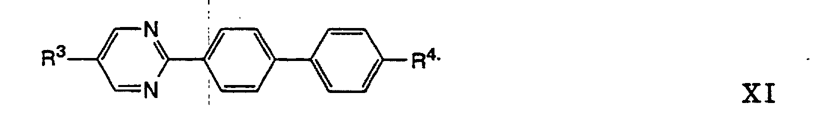 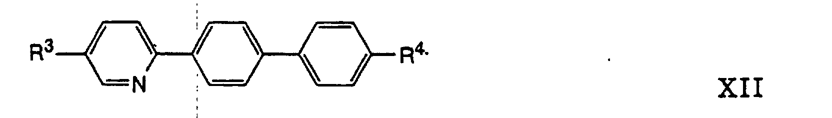 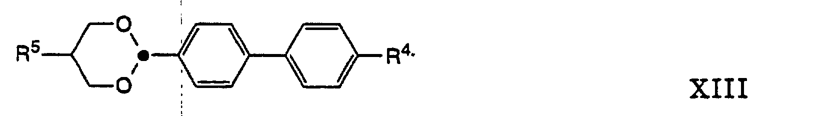 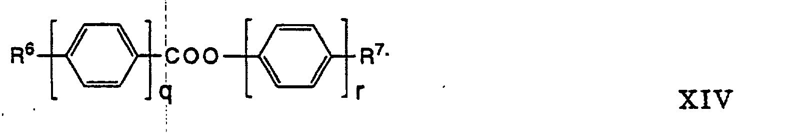 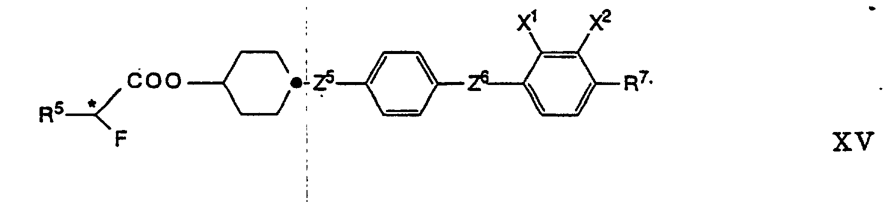 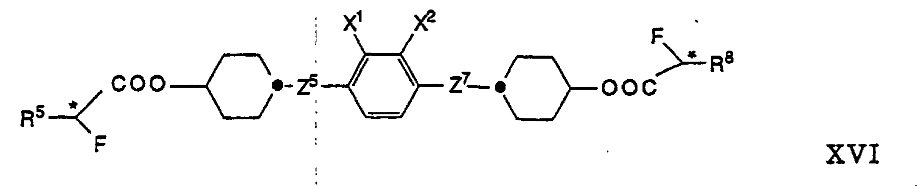 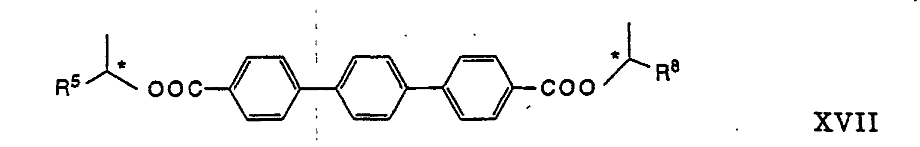 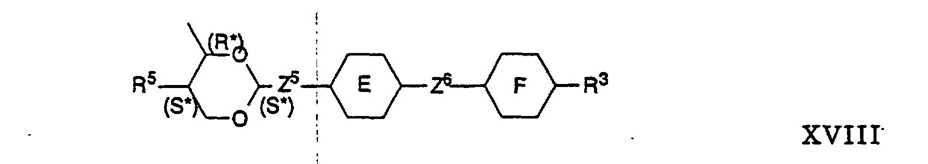 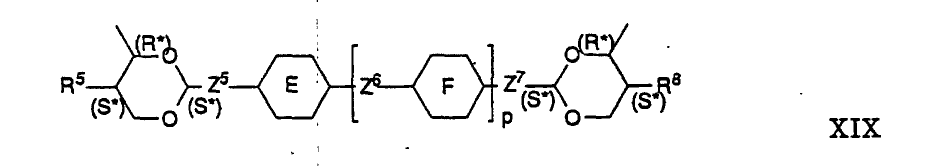 worin
- p: 0 oder 1,
- q und r: unabhängig voneinander 1 oder 2, wobei q + r = 2 oder 3;
- R³ und R⁴: unabhängig voneinander Alkyl, Alkenyl, Alkoxy, Alkenyloxy, Alkoxyalkyl, Alkoxyalkoxy, Alkanoyloxy, Alkenoyloxy, Alkoxycarbonyl, Alkenyloxycarbonyl;
- R⁵ und R⁸: unabhängig voneinander Alkyl oder Alkenyl;
- R⁶ und R⁷: unabhängig voneinander Alkyl, Alkenyl, Alkoxy und/oder Alkenyloxy;
- Z⁵, Z⁶ und Z⁷: unabhängig voneinander eine Einfachbindung, -CH₂CH₂- oder -CH₂O-, und Z⁶ auch -OCH₂-, -COO-oder -OOC-;
- E und F: unabhängig voneinander Phenylen-1,4-diyl oder trans-Cyclohexylen-1,4-diyl;
- X¹ und X²: unabhängig voneinander Wasserstoff oder Fluor; und
- (R*) und (S*): die relativen Konfigurationen bedeuten.

Die Verbindungen der Formeln XV bis XIX sind optisch aktive Dotierstoffe, welche, je nach gewünschtem Effekt neben einer oder mehreren Verbindungen der Formel I in einer der beiden enantiomeren Formen in der Mischung vorhanden sein können.

Die Substituenten R³ bis R⁸ besitzen höchstens je 18 Kohlenstoffatome, vorzugsweise 5-12 Kohlenstoffatome. Sie können geradkettig oder verzweigt sein, bevorzugt jedoch geradkettig.

Ebenfalls Gegenstand der vorliegenden Erfindung sind elektro-optische Vorrichtungen enthaltend eine oder mehrere Verbindungen der Formel I. Die Herstellung der flüssigkristallinen Gemische und der elektro-optischen Vorrichtung kann in an sich bekannter Weise erfolgen.

Die Herstellung der Verbindungen der Formel I sowie flüssigkristalliner Gemische enthaltend diese Verbindungen werden durch die folgenden Beispiele weiter veranschaulicht. Optische Antipoden der Verbindungen der Formel I besitzen jeweils die gleichen Phasenumwandlungstemperaturen und induzieren die gleichen Absolutwerte der spontanen Polarisation und der Verdrillung aber mit entgegengesetztem Vorzeichen. Die zur Charakterisierung der Phasenumwandlungen verwendeten Abkürzungen haben folgende Bedeutungen:
- C: kristallin,
- s: smektisch,
- S_{A}, S_{B}, S_{C} usw.: smektisch A, B, C usw.,
- S_{A}*, S_{F}* usw.: chiral smektisch C, F usw.
- N: nematisch,
- N*: cholesterisch, und
- I: isotrop.

### Beispiel 1

a) In einem Rundkolben mit Magnetrührer, Wasserabscheider und Kühler wurde unter Stickstoff ein Gemisch von 0,80 g 4-Cyano-4'-(trans-4-formylcyclohexyl)biphenyl (Herstellung s. Mol Cryst. Liq. Cryst. Vol. 131, 327 (1985)), 0,587 g (2R, 3S)-2-Octyl-1,3-butandiol (Herstellung s. EP-0 457 105) 40 ml Toluol und 8 Tropfen 2n Schwefelsäure während 15 Std. unter Rückfluss erhitzt. Dann wurde auf Raumtemperatur gekühlt, 30 Tropfen Triethylamin zugegeben und zwischen Diethylether und ges. Natriumbicarbonat-Lösung verteilt. Die organischen Phasen wurden abgetrennt hierauf über Magnesiumsulfat getrocknet, filtriert und das Filtrat eingedampft. Dies ergab 1,4 g rohes 4'-{trans-4-[(2R, 3S, 5R)-5-Octyl-4-methyl-1,3-dioxan-2-yl]cyclohexyl}-4-cyanobiphenyl als leicht gelblichen Festkörper.
b) 1,4 g 4'-{trans-4-[(2R, 3S, 5R)-5-Octyl-4-methyl-1,3-dioxan-2-yl]cyclohexyl}-4-cyanobiphenyl wurden in 30 ml Toluol gelöst, auf 0° C gekühlt, und innert 3 Min. tropfenweise mit 3,2 ml DIBAH-Lösung (20% in Toluol) versetzt. Das Reaktionsgemisch wurde auf Raumtemperatur erwärt und 15 Std. bei dieser Temperatur gerührt. Danach wurde zwischen Diethylether und Wasser verteilt, die Etherphasen auf 80 ml Wasser gegossen, die Mischung auf 0°C gekühlt und unter starkem Rühren tropfenweise mit 1N Salzsäure versetzt bis das pH 2 betrug. Das Gemisch wurde in einen Scheidetrichter transferiert, die wässrige Phase abgetrennt und die organische Phase sofort mit ges. Natriumbicarbonat-Lösung gewaschen. Die Etherphase wurde über Magnesiumsulfat getrocknet, filtriert und das Filtrat eingedampft. Dies ergab 1,37 g rohen 4'-{trans-4-[(2R, 3S, 5R)-5-Octyl-4-methyl-1,3-dioxan-2-yl]cyclohexyl}biphenyl-4-carboxaldehyd als gelben Festkörper.
c) In einem Rundkolben mit Magnetrührer, Wasserabscheider und Kühler wurde unter Stickstoff eine Lösung von 1,37 g 4'-{trans-4-[(2R, 3S, 5R)-5-Octyl-4-methyl-1,3-dioxan-2-yl]cyclohexyl}biphenyl-4-carboxaldehyd und 0,61 g (2R, 3S)-2-Octyl-1,3-butandiol in 40 ml Toluol mit 8 Tropfen 2N Schwefelsäure versetzt und während 2,5 Std. unter Rückfluss gerührt. Dann wurde auf Raumtemperatur gekühlt, 30 Tropfen Triethylamin zugefügt und das Reaktionsgemisch zwischen Diethylether und ges. Natriumcarbonat-Lösung verteilt. Die organischen Phasen wurden über Magnesiumsulfat getrocknet, filtriert, das Filtrat eingedampft und der Rückstand an 140 g Kieselgel mit Toluol/Essigester (50:1) chromatographiert. Dreimalige Kristallisation aus Hexan ergab 0,95 g reines 4'-{trans-4-[(2R, 3S, 5R)-5-Octyl-4-methyl-1,3-dioxan-2-yl]cyclohexyl}-4-[(2R, 3S, 5R)-5-octyl-4-methyl-1,3-dioxan-2-yl]biphenyl als farblose Kristalle, Smp. (C/S_{B}): 73,5 °C, S_{B}/S_{A}: 75,5 °C, S_{A}/N*: 106 °C, Klp. (N*/I): 219 °C, [α]_{D} (CHCl₃): -23,9°.

Auf analoge Weise können die folgenden Verbindungen hergestellt werden:
4'-{trans-4-[(2R, 3S, 5R)-5-Heptyl-4-methyl-1,3-dioxan-2-yl]cyclohexyl}-4-[(2R, 3S, 5R)-5-heptyl-4-methyl-1,3-dioxan-2-yl]biphenyl,
4'-{trans-4-[(2R, 3S, 5R)-5-Hexyl-4-methyl-1,3-dioxan-2-yl]cyclohexyl}-4-[(2R, 3S, 5R)-5-hexyl-4-methyl-1,3-dioxan-2-yl]biphenyl,
4'-{trans-4-[(2R, 3S, 5R)-5-nonyl-4-methyl-1,3-dioxan-2-yl]cyclohexyl}-4-[(2R, 3S, 5R)-5-nonyl-4-methyl-1,3-dioxan-2-yl]biphenyl,
2',3'-Difluor-4'-{trans-4-[(2R, 3S, 5R)-5-octyl-4-methyl-1,3-dioxan-2-yl]cyclohexyl}-4-[(2R, 3S, 5R)-5-octyl-4-methyl-1,3-dioxan-2-yl]biphenyl,
2,3-Difluor-4'-{trans-4-[(2R, 3S, 5R)-5-octyl-4-methyl-1,3-dioxan-2-yl]cyclohexyl}-4-[(2R, 3S, 5R)-5-heptyl-4-methyl-1,3-dioxan-2-yl]biphenyl,
4'-{2-{trans-4-[(2R, 3S, 5R)-5-Octyl-4-methyl-1,3-dioxan-2-yl]cyclohexyl}ethyl}-4-[(2R, 3S, 5R)-5-octyl-4-methyl-1,3-dioxan-2-yl]biphenyl,
4'-{trans-4-{2-[(2R, 3S, 5R)-5-Octyl-4-methyl-1,3-dioxan-2-yl]ethyl}cyclohexyl}4-[(2R, 3S, 5R)-5-octyl-4-methyl-1,3-dioxan-2-yl]biphenyl, Smp. (C/S_{A}): 88°C, S_{A}/N*: 98°C, Klp. (N*/I): 184,6°C,
4'-{{trans-4-[(2R, 3S, 5R)-5-Octyl-4-methyl-1,3-dioxan-2-yl]cyclohexyl}methoxy}-4-[(2R, 3S, 5R)-5-octyl-4-methyl-1,3-dioxan-2-yl]biphenyl,
4'-{2-{trans-4-[(2R, 3S, 5R)-5-Octyl-4-methyl-1,3-dioxan-2-yl]cyclohexyl}-ethyl}-4-{2-[(2R, 3S, 5R)-5-octyl-4-methyl-1,3-dioxan-2-yl]ethyl}biphenyl,
1,4-Bis-{trans-4-[(2R, 3S, 5R)-5-Octyl-4-methyl-1,3-dioxan-2-yl]cyclohexyl)benzol, Smp. (C/N*): 106,1°C, Klp. (N*/I): 222°C,
1,4-Bis-{2-{trans-4-[(2R, 3S, 5R)-5-Octyl-4-methyl-1,3-dioxan-2-yl]cyclohexyl}ethyl}benzol
1,4-Bis-{trans-4-(2-[(2R, 3S, 5R)-5-Octyl-4-methyl-1,3-dioxan-2-yl]ethyl}cyclohexyl}benzol

### Beispiel 2

a) In einem Rundkolben mit Magnetrührer, Wasserabscheider und Kühler wurde unter Stickstoff ein Gemisch von 0,871 g 4-Brombenzaldehyd, 0,587 g (2R, 3S)-2-Octyl-1,3-butandiol (Herstellung s. EP-A-0 457 105) 40 ml Toluol und 8 Tropfen 2n Schwefelsäure während 15 Std. unter Rückfluss erhitzt. Dann wurde auf Raumtemperatur abgekühlt, 30 Tropfen Triethylamin zugegeben und zwischen Diethylether und ges. Natriumbicarbonat-Lösung verteilt. Die organischen Phasen wurden vereinigt über Magnesiumsulfat getrocknet, filtriert und das Filtrat eingedampft. Chromatographie des Rückständes an 140 g Kieselgel mit 3% Essigester in Hexan ergab 0,69 g 1-Brom-4-[(2R, 3S, 5R)-5-Octyl-4-methyl-1,3-dioxan-2-yl]benzol als bräunliches Oel.
b) Ein Gemisch von 1,33 g 1-Brom-4-[(2R, 3S, 5R)-5-Octyl-4-methyl-1,3-dioxan-2-yl]benzol, 0,3 g Phenyl-1,4-bis-boronsäure, 0,077 g 10 proz. Palladium auf Kohle, 0,477 g fein gemahlenes Natriumcarbonat und 20 ml Ethanol wurden bei 90°C während 100 Std. gerührt. Dann wurden weitere 0,050 g Phenyl-1,4-bis-boronsäure, 0,02 g Katalysator und 0,2 g Natriumcarbonat zugefügt und während weiteren 15 Std. bei 90°C gerührt. Das Reaktionsgemisch wurde dann abgekühlt, über Celite/Kieselgel filtriert und das Filtrat zwischen Diethylether und IN Ammoniumchlorid-Lösung verteilt. Hierauf wurden die organischen Phasen vereinigt über Magnesiumsulfat getrocknet, filtriert und das Filtrat eingedampft. Chromatographie des Rückständes an 200 g Kieselgel mit 3% Essigester in Hexan und anschliessende Kristallisation aus Hexan ergab 0,1 g 1,4-Bis-{4-[(2R, 3S, 5R)-5-octyl-4-methyl-1,3-dioxan-2-yl]phenyl}benzol als farblose Kristalle, Smp.: < 25°C, S_{G}/S_{C}*: 129.8°C, S_{C}*/N*: 193°C, Klp. (N*/I): 246,3°C.

Auf analoge Weise können die folgenden Verbindungen hergestellt werden:
1,4-Bis-{4-[(2R, 3S, 5R)-5-heptyl-4-methyl-1,3-dioxan-2-yl]phenyl}benzol,
1,4-Bis-{4-[(2R, 3S, 5R)-5-hexyl-4-methyl-1,3-dioxan-2-yl]phenyl}benzol,
1,4-Bis-{4-[(2R, 3S, 5R)-5-pentyl-4-methyl-1,3-dioxan-2-yl]phenyl}benzol,
1,4-Bis-{4-[(2R, 3S, 5R)-5-nonyl-4-methyl-1,3-dioxan-2-yl]phenyl}benzol,
1,4-Bis-{4-[(2R, 3S, 5R)-5-decyl-4-methyl-1,3-dioxan-2-yl]phenyl}benzol,
1-{4-[(2R, 3S, 5R)-5-heptyl-4-methyl-1,3-dioxan-2-yl]phenyl}-4-{4-[(2R, 3S, 5R)-5-octyl-4-methyl-1,3-dioxan-2-yl]phenyl}benzol,
1,4-Bis-{4-[(2R, 3S, 5R)-5-(7-octenyl)-4-methyl-1,3-dioxan-2-yl]phenyl}-benzol,
1,4-Bis-{4-[(2R, 3S, 5R)-5-octyl-4-methyl-1,3-dioxan-2-yl]phenyl}-2,3-difluorbenzol,
1,4-Bis-{3-fluoro-4-[(2R, 3S, 5R)-5-(7-octenyl)-4-methyl-1,3-dioxan-2-yl]phenyl}benzol,
1,4-Bis-{2-fluoro-4-[(2R, 3S, 5R)-5-(7-octenyl)-4-methyl-1,3-dioxan-2-yl]phenyl}benzol,
1-{2,3Difluoro-4-[(2R, 3S, 5R)-5-(7-octenyl)-4-methyl-1,3-dioxan-2-yl]phenyl}-4-{4-[(2R, 3S, 5R)-5-(7-octenyl)-4-methyl-1,3-dioxan-2-yl]phenyl}benzol,
1,4-Bis-{4-{2-[(2R, 3S, 5R)-5-octyl-4-methyl-1,3-dioxan-2-yl]ethyl}phenyl]benzol, S/S_{A} 140,2°C, S_{A}/N* 175°C, Klp. (N*/I): 181,8°C.

### Beispiel 3

Zu einer Lösung von 2,05 g 4-{4-[(2R, 3S, 5R)-5-Octyl-4-methyl-1,3-dioxan-2-yl]phenyl}benzoesäure, 1,4 g 4-[(2R, 3S, 5R)-5-Octyl-4-methyl-1,3-dioxan-2-yl]phenol und 0,1 g 4-(Dimethylamino)pyridin in 250 ml Dichlormethan werden innert 10 Min. unter Rühren portionenweise 1,2 g N,N'-Dicyclohexylcarbodiimid gegeben. Das Gemisch wird über Nacht bei Raumtemperatur gerührt und dann filtriert. Das Filtrat wird mit Dichlormethan verdünnt, zweimal mit je 50 ml gesättigter Natriumcarbonat-Lösung und dann mit Wasser gewaschen, über Magnesiumsulfat getrocknet, filtriert und das Filtrat eingeengt. Chromatographie des Rückstandes an Kieselgel mit Toluol und Umkristallisation aus Hexan ergibt 4'-[(2R, 3S, 5R)-5-Octyl-4-methyl-1,3-dioxan-2-yl]biphenylcarbonsäure-4-[(2R, 3S, 5R)-5-octyl-4-methyl-1,3-dioxan-2-yl]phenylester: Smp. (C/S_{A}): 95.6°C, S_{A}/N* 106.6°C; Klp. (N*/I) 234°C.

Auf analoge Weise können die folgenden Verbindungen hergestellt werden:
4'-[(2R, 3S, 5R)-5-Octyl-4-methyl-1,3-dioxan-2-yl]biphenylcarbonsäure-4-[(2R, 3S, 5R)-5-nonyl-4-methyl-1,3-dioxan-2-yl]phenylester,
4'-[(2R, 3S, 5R)-5-Nonyl-4-methyl-1,3-dioxan-2-yl]biphenylcarbonsäure-4-[(2R, 3S, 5R)-5-hyptyl-4-methyl-1,3-dioxan-2-yl]phenylester,
4'-[(2R, 3S, 5R)-5-Octyl-4-methyl-1,3-dioxan-2-yl]biphenylcarbonsäure-4-[(2R, 3S, 5R)-5-(7-octenyl)-4-methyl-1,3-dioxan-2-yl]phenylester,
4'-[(2R, 3S, 5R)-5-(7-Octenyl)-4-methyl-1,3-dioxan-2-yl]biphenylcarbonsäure-4-[(2R, 3S, 5R)-5-octyl-4-methyl-1,3-dioxan-2-yl]phenylester,
2,3-Difluor-4'-[(2R, 3S, 5R)-5-octyl-4-methyl-1,3-dioxan-2-yl]biphenyl-carbonsäure-4-[(2R, 3S, 5R)-5-octyl-4-methyl-1,3-dioxan-2-yl]phenylester,
2',3'-Difluor-4'-[(2R, 3S, 5R)-5-octyl-4-methyl-1,3-dioxan-2-yl]biphenyl-carbonsäure-4-[(2R, 3S, 5R)-5-octyl-4-methyl-1,3-dioxan-2-yl]phenylester,
4-[(2R, 3S, 5R)-5-Octyl-4-methyl-1,3-dioxan-2-yl]benzoesäure-4'-[(2R,3S, 5R)-5-octyl-4-methyl-1,3-dioxan-2-yl]biphenylester, Smp. (C/S_{A}): 114,1°C, S_{A}/N*: 128,5°C, Klp. (N*/I): 230,6°C,
4-[(2R, 3S, 5R)-5-Octyl-4-methyl-1,3-dioxan-2-yl]benzoesäure-4-(trans-4-[(2R, 3S, 5R)-5-octyl-4-methyl-1,3-dioxan-2-yl]cyclohexyl}phenylester,
4-{trans-4-[(2R, 3S, 5R)-5-Octyl-4-methyl-1,3-dioxan-2-yl]cyclohexyl}-benzoesäure-4-[(2R, 3S, 5R)-5-octyl-4-methyl-1,3-dioxan-2-yl]phenylester.

### Beispiel 4

Zur Untersuchung der Eigenschaften der Verbindungen der Formel I in Gemischen wurden Testmischungen je mit den beiden Grundmischungen GM-I und GM-II hergestellt. GM-I ist eine achirale (undotierte) Mischung und diente zur Ermittlung der Phasenfolge, insbesondere der Höhe des S_{C}*-Phasenüberganges, sowie der spontanen Polarisation (Ps). GM-II ist eine chiral dotierte Mischung und diente der Messung der Wellenlänge der selektiven Reflexion (λₛₑₗ). Diese ist ein Mass für die Ganghöhe der Helix, d.h. je kleiner der Messwert für λₛₑₗ desto kleiner ist die Ganghöhe (Pitch) und desto stärker das Verdrillungsvermögen des Dotierstoffes. Zu Vergleichszwecken wurden zudem zwei Vergleichsmischungen hergestellt. Dazu wurden die beiden Grundmischungen GM-I und GM-II je mit einem vierkernigen chiralen Bismethyldioxan dotiert.

Die Messungen wurden unter den folgenden Bedingungen durchgeführt: Ps- Messungen bei ca. 8 mµ Zelldicke und einer Dreieckspannung von 10 Hz und 5 V/µ. Messungen von λ_{sel.} bei homöotroper Orientierung. Für alle Messung wurde eine Temperatur von 25 °C eingehalten.

| Grundmischung I (GM-I) | |
|---|---|
| 7,0 Gew.% | 5-(5-Heptyl-1,3-dioxan-2-yl)-2-(4-octyloxyphenyl)pyridin; |
| 7,0 Gew.% | 5-(5-Octyl-1,3-dioxan-2-yl)-2-(4-octyloxyphenyl)pyridin; |
| 6,0 Gew.% | 5-(5-Decyl-1,3-dioxan-2-yl)-2-(4-octylphenyl)pyridin; |
| 15,9 Gew.% | 4-Decyloxybenzoesäure-4-[2-(trans-4-pentylcyclohexyl)-ethyl]phenylester; |
| 7,1 Gew.% | 4-Dodecyloxybenzoesäure-4-[2-(trans-4-pentylcyclohexyl)-ethyl]phenylester; |
| 7,0 Gew.% | 2,3-Difluoro-4-undecyloxybenzoesäure-4-[2-(trans-4-pentylcyclohexyl)ethyl]phenylester; |
| 14,9 Gew.% | 2-(4-Hexyloxyphenyl)-5-nonylpyrimidin; |
| 19,9 Gew.% | 2-(4-Nonyloxyphenyl)-5-nonylpyrimidin; |
| 5,0 Gew.% | 2-(4-Octyloxyphenyl)-5-heptylpyrimidin; |
| 10,1 Gew.% | 2-(4-Hexyloxyphenyl)-5-octylpyrimidin; |
| Phasenfolge: | I - 103°C - N - 80°C - S_{A} - 76,5°C - S_{C} - -17,5°C C |

| Vergleichsmischung I | |
|---|---|
| 7,0 Gew.% | 1,4-Bis-{4-[(2R, 3S, 5R)-5-octyl-4-methyl-1,3-dioxan-2-yl]}benzol |
| 93,0 Gew.% | GM-I |
| Phasenfolge: | I - 104,0°C - N* - 67,4°C - S_{C}* - ; |
| Ps: | 10,1 nC/cm² |

| Testmischung I-1 | |
|---|---|
| 7,0 Gew.% | 1,4-Bis-{4-[(2R, 3S, 5R)-5-octyl-4-methyl-1,3-dioxan-2-yl]phenyl}benzol |
| 93,0 Gew.% | GM-I |
| Phasenfolge: | I - 109,4°C - N* - 80°C - S_{A} - 72,9°C - S_{C}* -; |
| Ps: | 13,1 nC/cm² |

| Testmischung I-2 | |
|---|---|
| 7,0 Gew.% | 4'-{trans-4-[(2R, 3S, 5R)-5-Octyl-4-methyl-1,3-dioxan-2-yl]cyclohexyl}-4-[(2R, 3S, 5R)-5-octyl-4-methyl-1,3-dioxan-2-yl]biphenyl |
| 93,0 Gew.% | GM-I |
| Phasenfolge: | I - 110,1°C - N* - 72,0°C - S_{C}* - ; |
| Ps: | 11,5 nC/cm² |

| Grundmischung II (GM-II) | |
|---|---|
| 27,0 Gew.% | 1,4-Bis-{4-[(S)-2-Octyloxy]phenyl}benzol |
| 15,7 Gew.% | 2-(4-Octyloxyphenyl)-5-[(4E)-4-octenoyloxy]pyrimidin; |
| 5,2 Gew.% | 2-(4-Octanoyloxyphenyl)-5-octylpyridin; |
| 5,2 Gew.% | 2-{4-[(2E)-2-Octenoyloxy]phenyl]-5-heptylpyridin; |
| 5,2 Gew.% | 2-{4-[(2E)-2-Qctenoyloxy]phenyl}-5-octylpyridin; |
| 10,4 Gew.% | 2-(4-Heptylphenyl)-5-[(2E)-2-octenyloxy]pyrimidin; |
| 15,6 Gew.% | 2-(4-Hexyloxyphenyl)-5-nonylpyrimidin; |
| 15,6 Gew.% | 2-(4-Nonyloxyphenyl)-5-nonylpyrimidin; |
| λₛₑₗ: | 508 nm |

| Vergleichsmischung II | |
|---|---|
| 3,0 Gew.% | 1,4-Bis-{4-[(2R, 3S, 5R)-5-octyl-4-methyl-1,3-dioxan-2-yl]}-benzol |
| 93,0 Gew.% | GM-I |
| λₛₑₗ: | 437 nm |

| Testmischung II-1 | |
|---|---|
| 3,0 Gew.% | 1,4-Bis-{4-[(2R, 3S, 5R)-5-octyl-4-methyl-1,3-dioxan-2-yl]-phenyl}benzol |
| 97,0 Gew.% | GM-II |
| λₛₑₗ: | 404 nm |

| Testmischung II-2 | |
|---|---|
| 3,0 Gew.% | 4'-{trans-4-[(2R, 3S, 5R)-5-Octyl-4-methyl-1,3-dioxan-2-yl]-cyclohexyl}-4-[(2R, 3S, 5R)-5-octyl-4-methyl-1,3-dioxan-2-yl]-biphenyl |
| 97,0 Gew.% | GM-II |
| λₛₑₗ: | 414 nm |

## Patentansprüche

1. Verbindungen der allgemeinen Formel 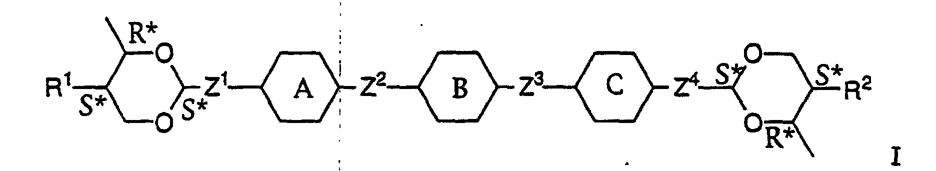 worin
R¹ und R² unabhängig von einander Alkyl oder Alkenyl, in welchen gegebenenfalls eine oder zwei nicht benachbarte Methylengruppen durch -O-, -COO-und/oder -OOC- ersetzt sind und/oder eines oder mehrere Wasserstoffatome durch Fluor, Chlor oder Cyano ersetzt sind, bedeuten; einer der Ringe
A, B und C ein gegebenenfalls mit 1 oder 2 Fluoratomen substituiertes 1,4-Phenylen, Pyridin-2,5-diyl oder Pyrimidin-2,5-diyl, und die beiden anderen Ringe unabhängig voneinander gegebenfalls mit 1 oder 2 Fluoratomen substituiertes 1,4-Phenylen oder trans-1,4-Cyclohexylen bedeuten;
Z¹ und Z⁴ unabhängig voneinander eine Einfachbindung, -CH₂CH₂-, -CH₂O-, -OCH₂-, -C≡C-, -(CH₂)₄-, -(CH₂)₃O-, -O(CH₂)₃-, -CH=CH-CH₂O-, -OCH₂-CH=CH-, -CH=CH-(CH₂)₂- oder -(CH₂)₂CH=CH-, mit der Massgabe, dass das Sauerstoffatom nicht direkt mit dem chiralen Dioxanring verknüpft ist, bedeuten;
Z² und Z³ unabhängig voneinander eine Einfachbindung, -CH₂-CH₂-, -O-CH₂-, -CH₂-O-, -COO-, -OOC-, -C≡C-, -(CH₂)₄-, -O(CH₂)₃-, -(CH₂)₃O-,-CH=CH-CH₂O-, -OCH₂-CH=CH-, -(CH₂)₂-CH=CH- oder -CH=CH-(CH₂)₂- bedeuten; und
R* und S* die relativen Konfigurationen der chiralen Kohlenstoffatome bedeuten.

2. Verbindungen nach Anspruch 1, dadurch gekennzeichnet, dass Z¹ und Z⁴ eine Einfachbindung, -CH₂CH₂-, -CH₂O- oder -OCH₂-; und Z² und Z³ eine Einfachbindung, -CH₂CH₂-, -CH₂O-, -OCH₂-, -COO- oder -OOC-bedeuten, wobei ein in den Gruppen Z¹ bis Z⁴ vorhandenes Sauerstoffatom vorzugsweise mit einem aromatischen Ring verknüpft ist.

3. Verbindungen nach einem der Ansprüche 1 oder 2, dadurch gekennzeichnet, dass höchstens zwei der Gruppen Z¹ bis Z⁴ verschieden von einer Einfachbindung sind.

4. Verbindungen nach einem der Ansprüche 1 bis 3 der allgemeinen Formeln 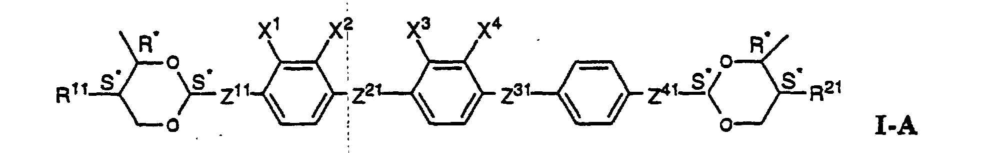 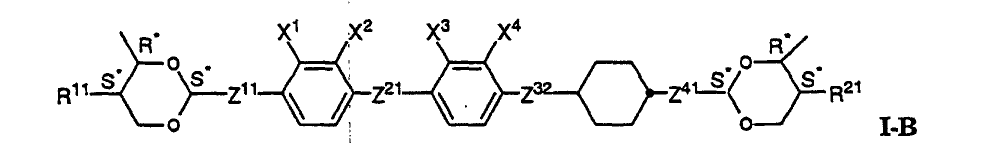 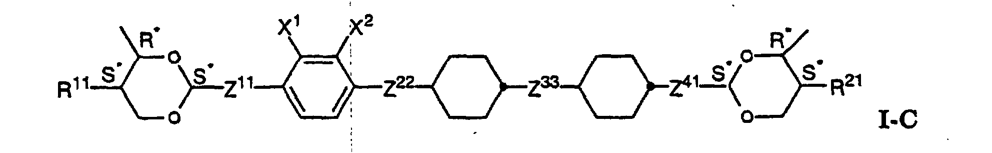 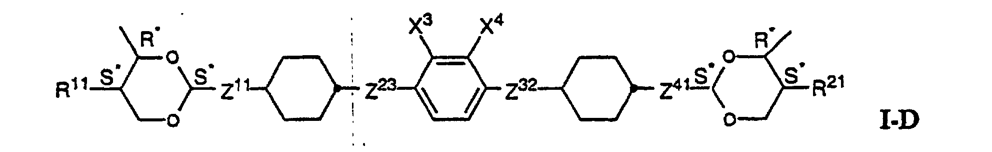 worin
R¹¹ und R²¹ unabhängig voneinander Alkyl oder Alkenyl mit höchstens 12 Kohlenstoffatomen, in welchen gegebenenfalls eine Methylengruppe durch -O-, -COO-oder -OOC- ersetzt ist;
Z¹¹, Z³³ und Z⁴¹ unabhängig voneinander eine Einfachbindung oder -CH₂CH₂-;
Z²¹, Z³¹ eine Einfachbindung, -COO- oder -OOC-;
Z²², Z³² unabhängig voneinander eine Einfachbindung, -CH₂CH₂- oder -OCH₂-;
Z²³ eine Einfachbindung, -CH₂CH₂- oder -CH₂O-; und die Substituenten
X¹ bis X⁴ unabhängig voneinander Wasserstoff oder Fluor bedeuten.

5. Verbindungen nach Anspruch 4 der Formeln 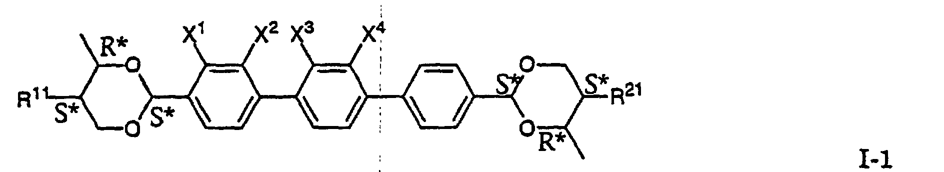 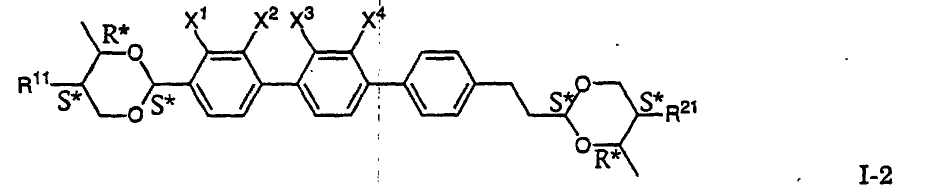 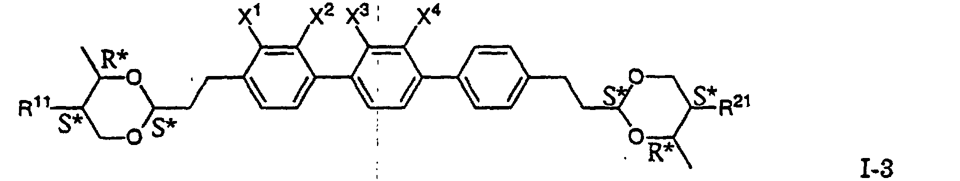 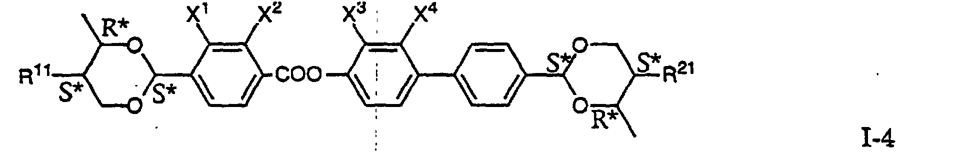 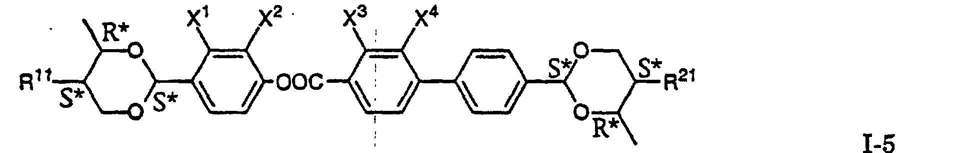 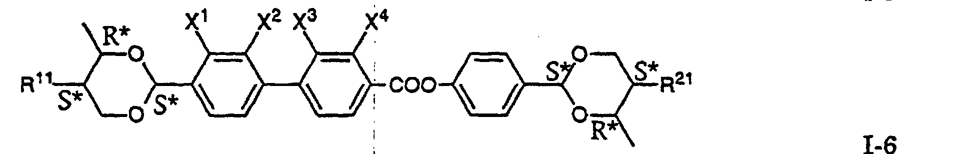 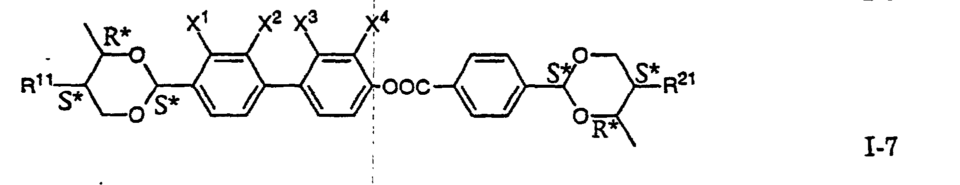 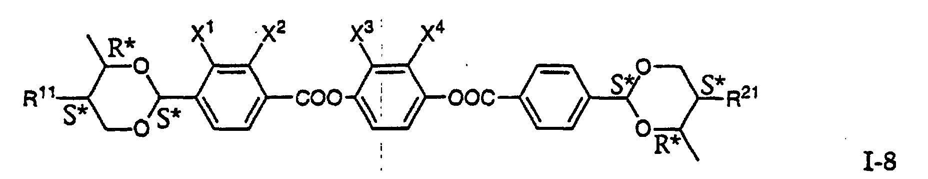 worin
R¹¹ und R²¹ unabhängig voneinander Alkyl oder Alkenyl mit höchstens 12 Kohlenstoffatomen, in welchen gegebenenfalls eine Methylengruppe durch -O-, -COO-oder -OOC- ersetzt ist; und die Substituenten
X¹ bis X⁴ unabhängig voneinander Wasserstoff oder Fluor bedeuten.

6. Verbindungen nach Anspruch 4 der Formeln 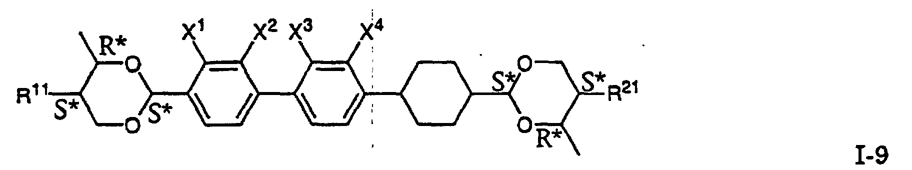 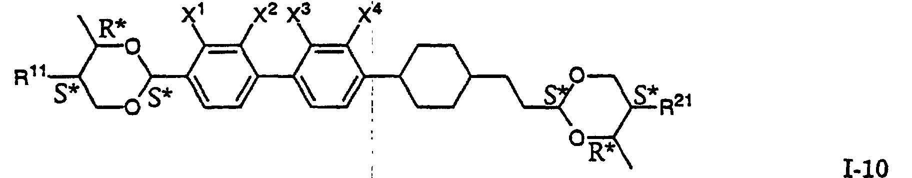 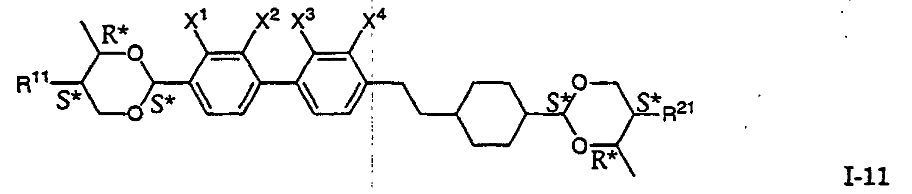 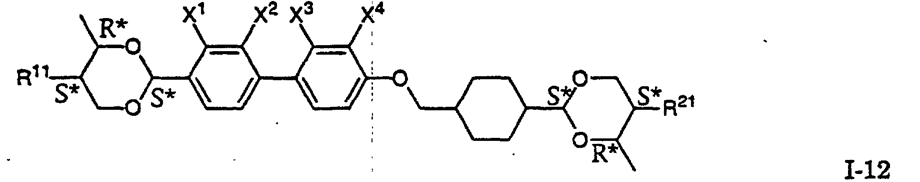 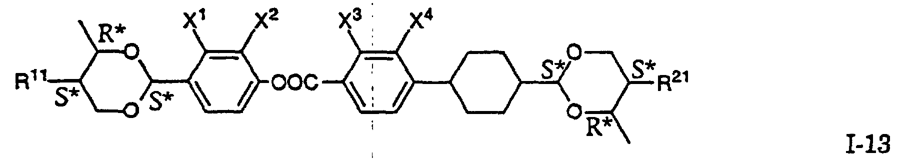 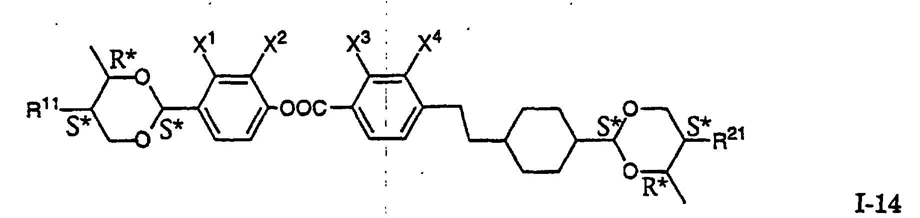 worin
R¹¹ und R²¹ unabhängig voneinander Alkyl oder Alkenyl mit höchstens 12 Kohlenstoffatomen, in welchen gegebenenfalls eine Methylengruppe durch -O-, -COO-oder -OOC- ersetzt ist; und die Substituenten
X¹ bis X⁴ unabhängig voneinander Wasserstoff oder Fluor bedeuten.

7. Verbindungen nach Anspruch 4 der Formeln 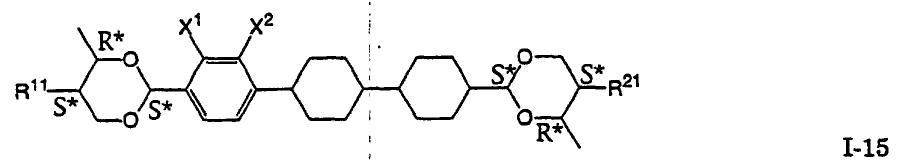 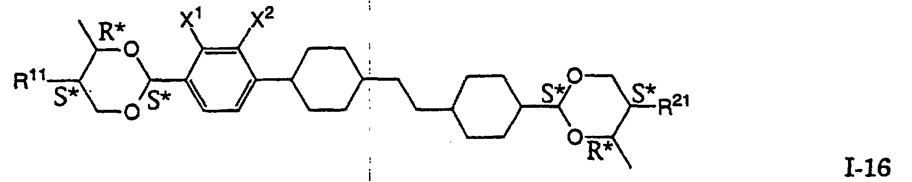 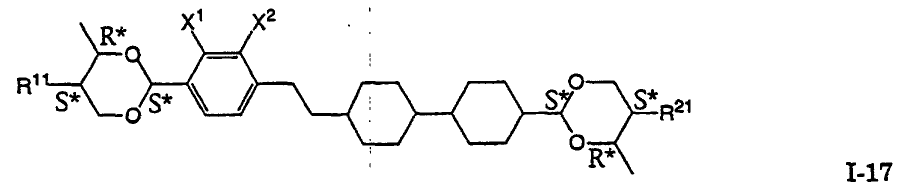 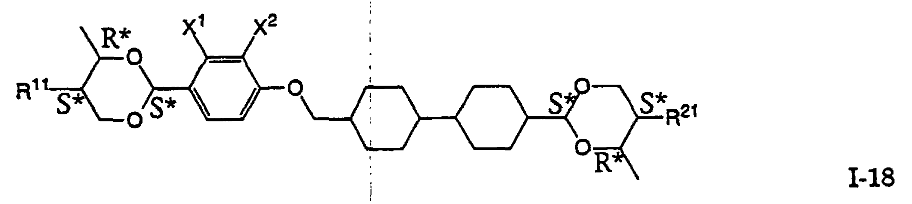 worin
R¹¹ und R²¹ unabhängig voneinander Alkyl oder Alkenyl mit höchstens 12 Kohlenstoffatomen, in welchen gegebenenfalls eine Methylengruppe durch -O-, -COO-oder -OOC- ersetzt ist; und die Substituenten
X¹ bis X⁴ unabhängig voneinander Wasserstoff oder Fluor bedeuten.

8. Verbindungen nach Anspruch 4 der Formeln 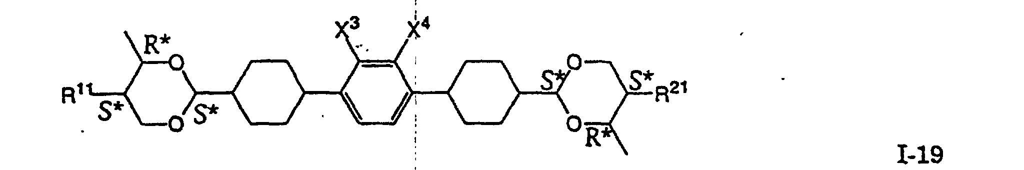 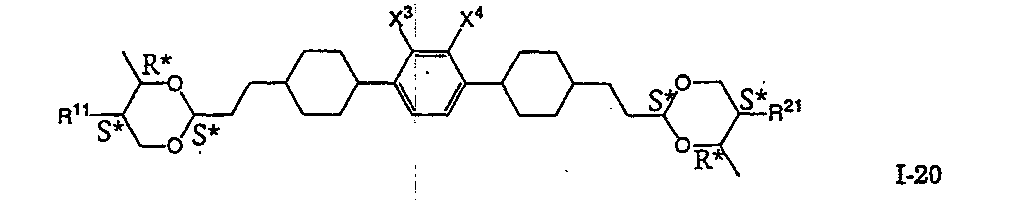 worin
R¹¹ und R²¹ unabhängig voneinander Alkyl oder Alkenyl mit höchstens 12 Kohlenstoffatomen, in welchen gegebenenfalls eine Methylengruppe durch -O-, -COO-oder -OOC- ersetzt ist; und die Substituenten
X¹ bis X⁴ unabhängig voneinander Wasserstoff oder Fluor bedeuten.

9. Verbindungen nach einem der Ansprüche 4 bis 8, dadurch gekennzeichnet, dass höchstens zwei der Gruppen X¹ bis X⁴ Fluor bedeuten.

10. Flüssigkristalline Gemische enthaltend mindestens zwei Komponenten, dadurch gekennzeichnet, dass mindestens eine Komponente eine optisch aktive Verbindung gemäss einem der Ansprüche 1 bis 9 ist.

11. Flüssigkristalline Gemische gemäss Anspruch 10, dadurch gekennzeichnet, dass der Anteil an optisch aktiven Verbindungen gemäss einem der Ansprüche 1 bis 9 1-30 Gew.-% beträgt.

12. Verwendung von Verbindungen nach einem der Ansprüche 1 bis 9 für elektro-optische Zwecke.

13. Verwendung von flüssigkristallinen Gemischen gemäss einem der Ansprüche 10 oder 11 für elektro-optische Zwecke.

14. Elektro-optische Vorrichtungen enthaltend Verbindungen gemäss einem der Ansprüche 1-9.

15. Elektro-optische Vorrichtungen enthaltend Mischungen gemäss einem der Ansprüche 10 oder 11.

## Claims

1. Compounds of the general formula 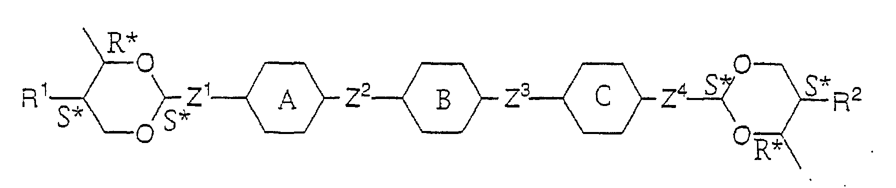 wherein
R¹ and R² each independently signify alkyl or alkenyl in which optionally one methylene group or two non-adjacent methylene groups is/are replaced by -0-, -COO- and/or -OOC-and/or one or more hydrogen atoms is/are replaced by fluorine, chlorine or cyano; one of rings
A, B and C signifies a 1,4-phenylene optionally substituted with 1 or 2 fluorine atoms, pyridine-2,5-diyl or pyrimidine-2,5-diyl and the other two rings each independently signify 1,4-phenylene optionally substituted with 1 or 2 fluorine atoms or trans-1,4-cyclohexylene;
Z¹ and Z⁴ each independently signify a single bond, -CH₂CH₂-, -CH₂O-, -OCH₂-, -C≡C-, -(CH₂)₄-, -(CH₂)₃O-, -O(CH₂)₃-, -CH=CH-CH₂O-, -OCH₂-CH=CH-, -CH=CH-(CH₂)₂- or -(CH₂)₂CH=CH-, with the proviso that the oxygen atom is not linked directly with the chiral dioxane ring;
Z² and Z³ each independently signify a single bond, -CH₂-CH₂-, -O-CH₂-, -CH₂-O-, -COO-, -OOC-, -C≡C-, -(CH₂)₄-, -O(CH₂)₃-, -(CH₂)₃O-, -CH=CH-CH₂O-, -OCH₂-CH=CH-, -(CH₂)₂-CH=CH- or -CH=CH-(CH₂)₂-; and
R*.and S* signify the relative configurations of the chiral carbon atoms.

2. Compounds according to claim 1, wherein Z¹ and Z⁴ signify a single bond, -CH₂CH₂-, -CH₂O- or -OCH₂-; and Z² and Z³ signify a single bond, -CH₂CH₂-, -CH₂O-, -OCH₂-, -COO- or -OOC-, with an oxygen atom present in groups Z¹ to Z⁴ preferably being linked with an aromatic ring.

3. Compounds according to claim 1 or claim 2, wherein a maximum of two of groups Z¹ to Z⁴ are other than a single bond.

4. Compounds according to any one of claims 1 to 3 of the general formulae 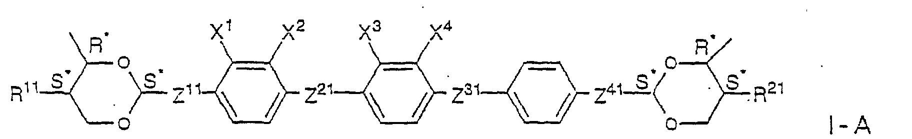 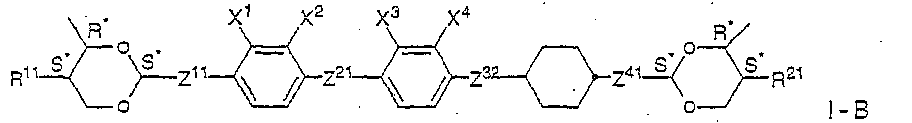 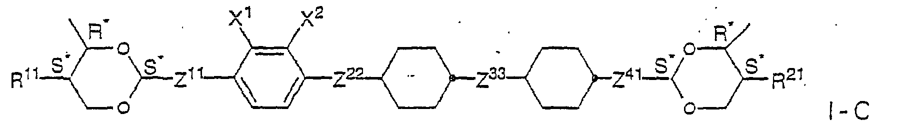 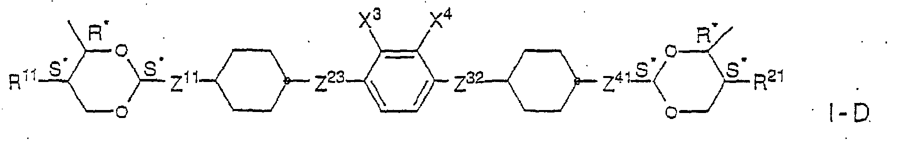 wherein
R¹¹ and R²¹ each independently signify alkyl or alkenyl with a maximum of 12 carbon atoms in which optionally one methylene group is replaced by -O-, -COO- or -OOC-;
Z¹¹,Z³³ and Z⁴¹ each independently signify a single bond, or -CH₂CH₂-;
Z²¹ and Z³¹ each independently signify a single bond, -COO- or -OOC-;
Z²² and Z³² each independently signify a single bond, -CH₂CH₂- or -OCH₂-;
Z²³ signifies a single bond, -CH₂CH₂- or -CH₂O-; and the substituents
X¹ to X⁴ each independently signify hydrogen or fluorine.

5. Compounds according to claim 4 of the formulae 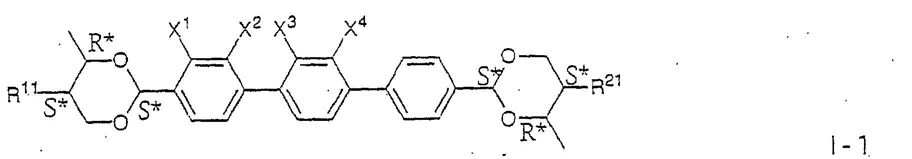 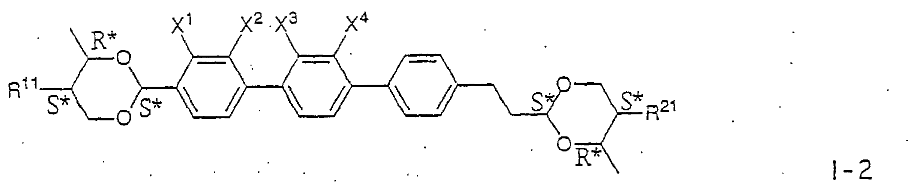 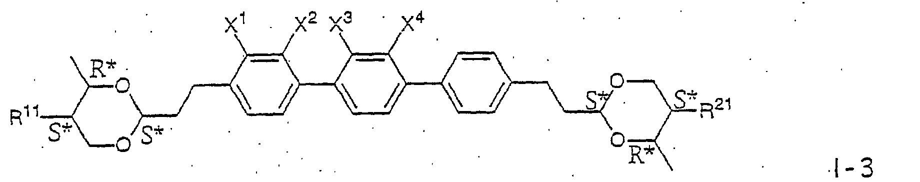 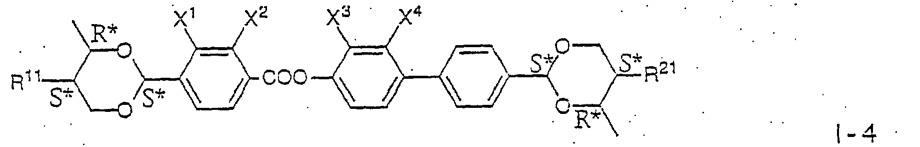 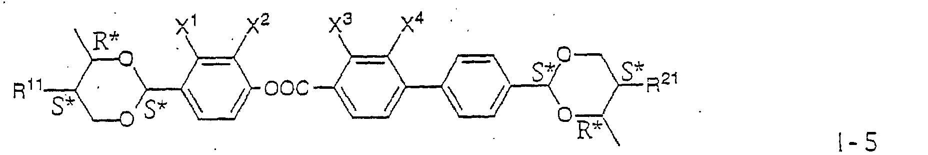 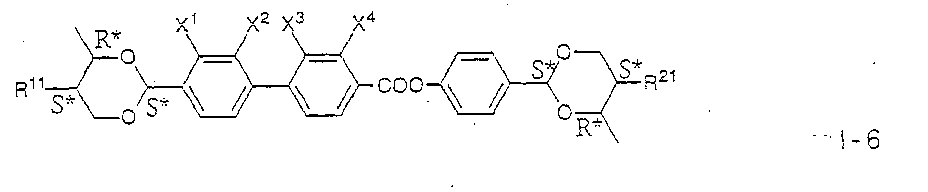 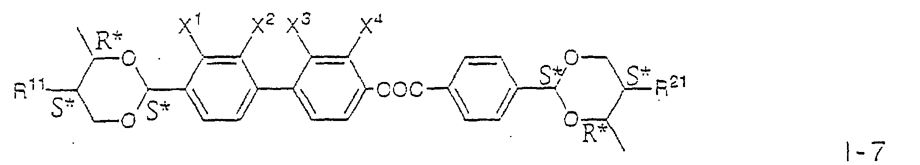 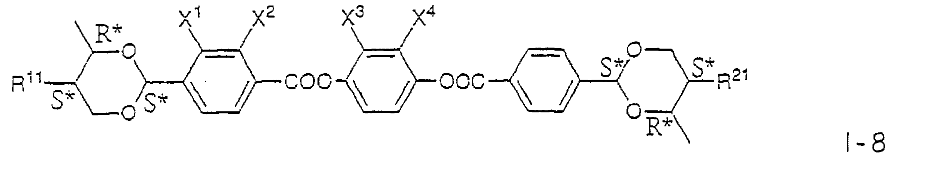 wherein
R¹¹ and R²¹ each independently signify alkyl or alkenyl with a maximum of 12 carbon atoms in which optionally one methylene group is replaced by -O-, -COO- or -OOC-; and the substituents
X¹ to X⁴ each independently signify hydrogen or fluorine.

6. Compounds according to claim 4 of the formulae 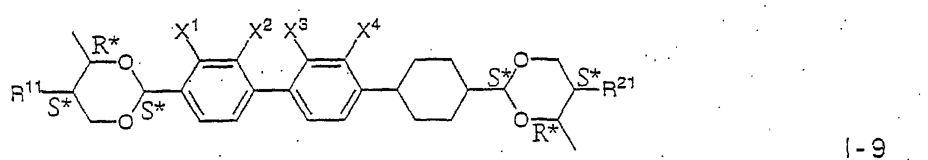 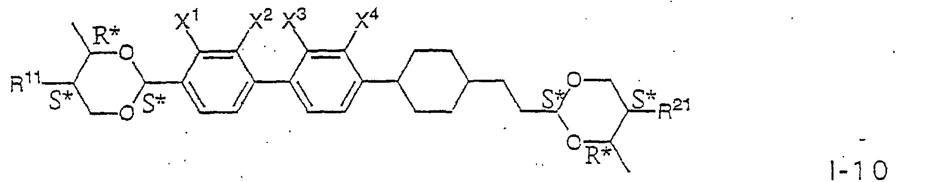 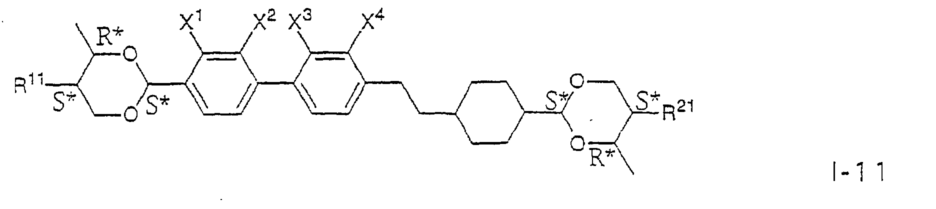 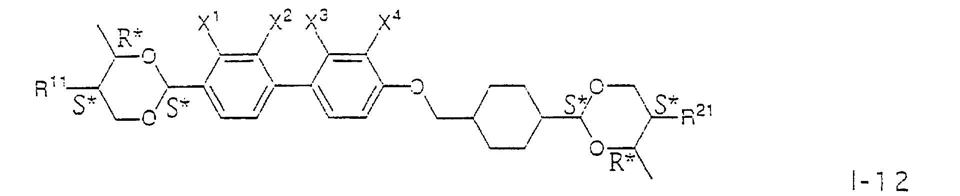 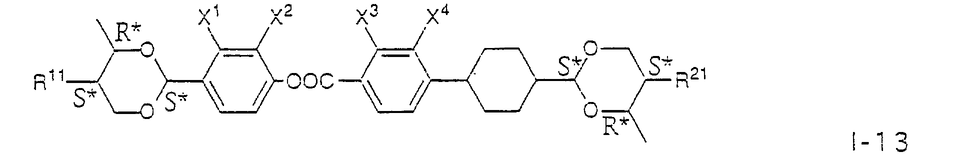 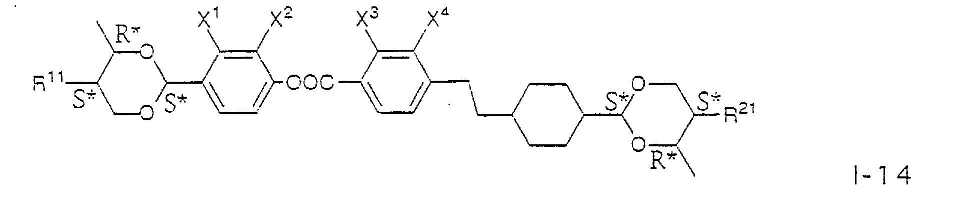 wherein
R¹¹ and R²¹ each independently signify alkyl or alkenyl with a maximum of 12 carbon atoms in which optionally one methylene group is replaced by -O-, -COO- or -OOC-; and the substituents
X¹ to X⁴ each independently signify hydrogen or fluorine.

7. Compounds according to claim 4 of the formulae 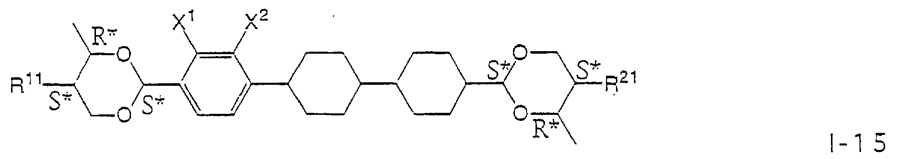 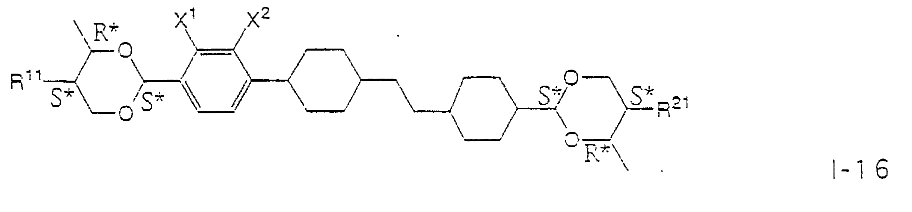 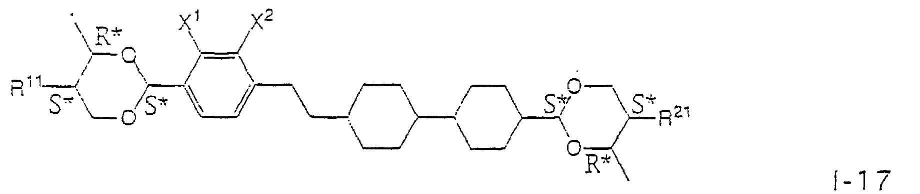 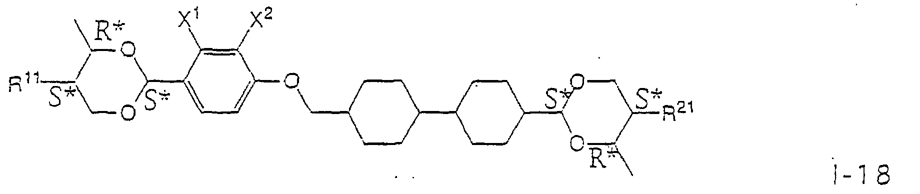 wherein
R¹¹ and R²¹ each independently signify alkyl or alkenyl with a maximum of 12 carbon atoms in which optionally one methylene group is replaced by -0-, -COO- or -OOC-; and the substituents
X¹ to X⁴ each independently signify hydrogen or fluorine.

8. Compounds according to claim 4 of the formulae 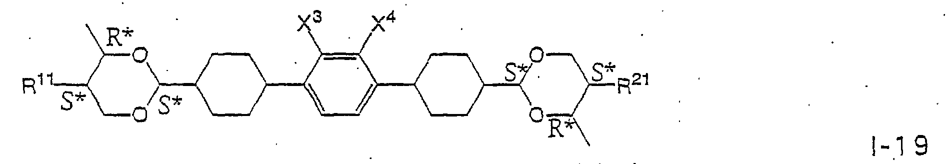 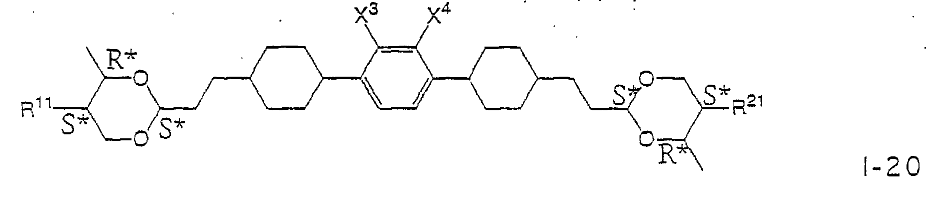 wherein
R¹¹ and R²¹ each independently signify alkyl or alkenyl with a maximum of 12 carbon atoms in which optionally one methylene group is replaced by -O-, -COO- or -OOC-; and the substituents
X¹ to X⁴ each independently signify hydrogen or fluorine.

9. Compounds according to any one of claims 4 to 8, wherein a maximum of two of groups X¹ to X⁴ signify fluorine.

10. Liquid crystalline mixtures containing at least two components, wherein at least one component is an optically active compound of formula I defined in anyone of claims 1 to 9.

11. Liquid crystalline mixtures in accordance with claim 10, wherein the content of optically active compounds defined in anyone of claims 1 to 9 is 1-30 wt%.

12. The use of the compounds defined in anyone of claims 1 to 9 for electro-optical purposes.

13. The use of liquid crystalline mixtures in accordance with claim 10 or claim 11 for electro-optical purposes.

14. An electro-optical device containing a compound according to any one of claims 1 to 9.

15. An electro-optical device containing a mixture according to claim 10 or claim 11.

## Revendications

1. Composés de formule générale 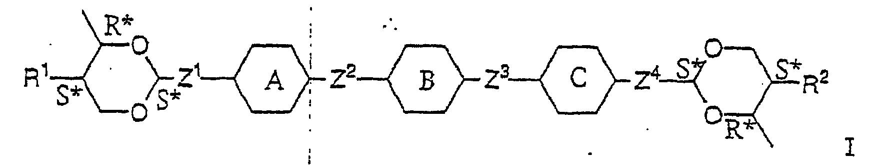 dans laquelle
R¹ et R² représentent indépendamment l'un de l'autre un groupe alkyle ou alcényle, dans lequel éventuellement un ou deux groupes méthylène non voisins sont remplacés par -O-, -COO- et/ou -OOC- et/ou un ou plusieurs atomes d'hydrogène sont remplacés par le fluor, le chlore ou un cyano ; un des cycles
A, B et C représentent un groupe 1,4-phénylène éventuellement substitué par 1 ou 2 atomes de fluor, pyridin-2,5-diyle ou pyrimidin-2,5-diyle, et les deux autres cycles représentent indépendamment l'un de l'autre un groupe 1,4-phénylène éventuellement substitué par un ou 2 atomes de fluor ou trans-1,4-cyclohexylène;
Z¹ et Z⁴ représentent indépendamment l'un de l'autre une liaison simple, -CH₂CH₂-, -CH₂O-, -OCH₂-, -C≡C-, -(CH₂)₄-, -(CH₂)₃O-, -O(CH₂)₃-, -CH=CH-CH₂O-, -OCH₂-CH=CH-, -CH=CH-(CH₂)₂- ou -(CH₂)₂-CH=CH-, sous réserve que l'atome d'oxygène ne soit pas relié directement au noyau dioxane chiral ;
Z² et Z³ représentent indépendamment l'un de l'autre une liaison simple, -CH₂CH₂-, -O-CH₂-, -CH₂-O-, -COO-, -OOC-, -C≡C-, -(CH₂)₄-, -O(CH₂)₃-, -(CH₂)₃O-,-CH=CH-CH₂O-, -OCH₂-CH=CH-, -(CH₂)₂-CH=CH- ou -CH=CH-(CH₂)₂- ; et
R* et S* représentent les configurations relatives des atomes de carbone chiraux.

2. Composés selon la revendication 1, caractérisés en ce que Z¹ et Z⁴ représentent une liaison simple, -CH₂CH₂-, -CH₂O- ou -OCH₂- ; et Z² et Z³ représentent une liaison simple, -CH₂CH₂-, -CH₂O-, -OCH₂-, -COO- ou -OOC-, un atome d'oxygène présent dans les groupes Z¹ à Z⁴ étant relié de préférence à un noyau aromatique.

3. Composés selon l'une des revendications 1 ou 2, caractérisés en ce qu'au plus deux des groupes Z¹ à Z⁴ sont différents d'une liaison simple.

4. Composés selon l'une des revendications 1 à 3 ayant les formules générales 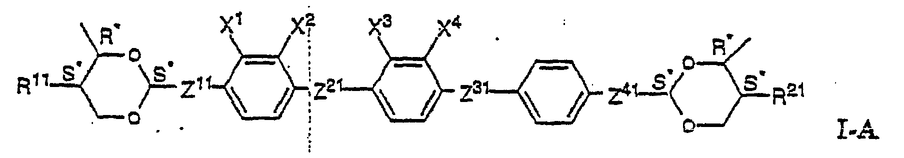 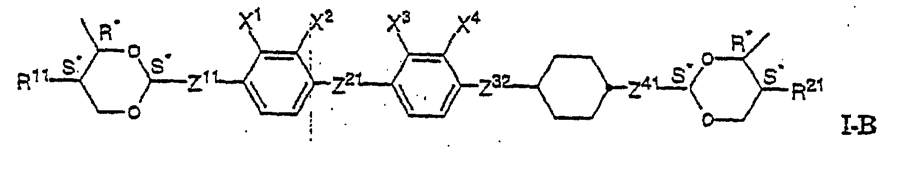 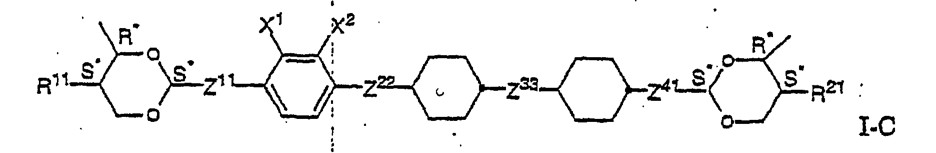 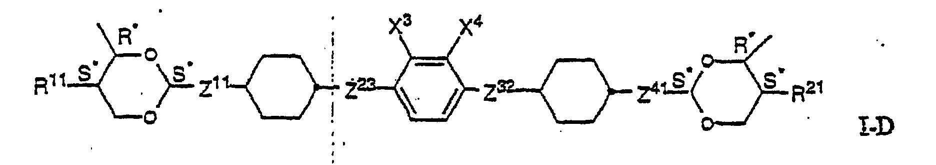 dans lesquelles
R¹¹ et R²¹ représentent indépendamment l'un de l'autre un alkyle ou alcényle ayant au plus 12 atomes de carbone, dans lequel éventuellement un groupe méthylène est remplacé par -O-, -COO- ou -OOC- ;
Z¹¹, Z²³ et Z⁴¹ représentent indépendamment l'un de l'autre une liaison simple ou -CH₂CH₂- ;
Z²¹, Z³¹ représentent une liaison simple, -COO- ou -OOC- ;
Z²², Z³² représentent indépendamment l'un de l'autre une liaison simple, -CH₂CH₂- ou -OCH₂- ;
Z²³ représente une liaison simple, -CH₂CH₂- ou -CH₂O- ; et les substituants
X¹ à X⁴ représentent indépendamment les uns des autres un atome d'hydrogène ou de fluor.

5. Composés selon la revendication 4 ayant les formules 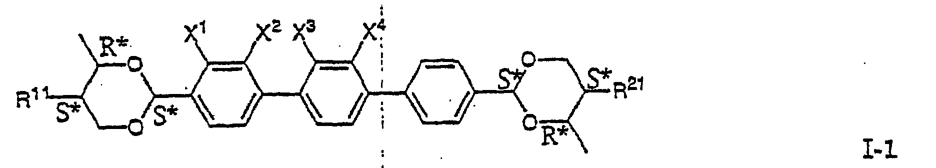 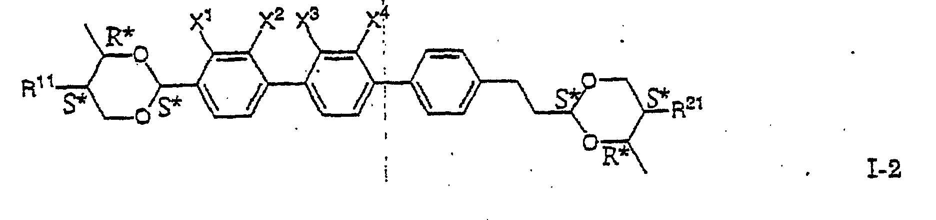 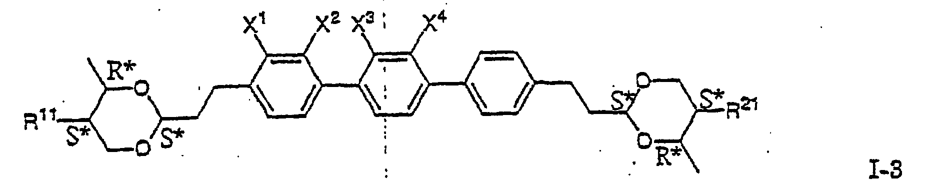 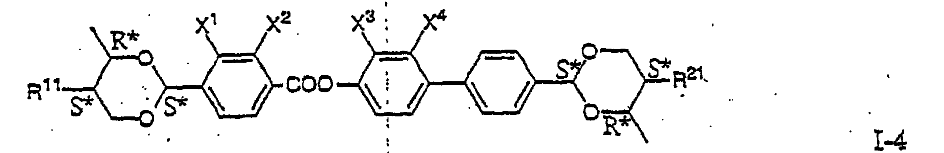 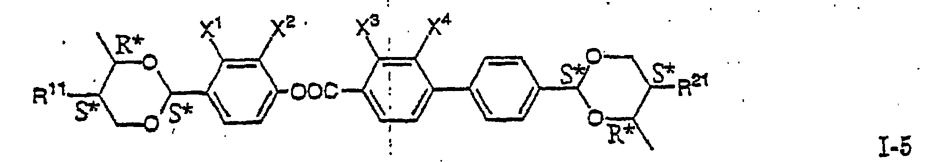 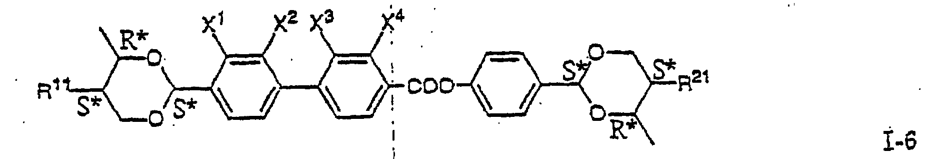 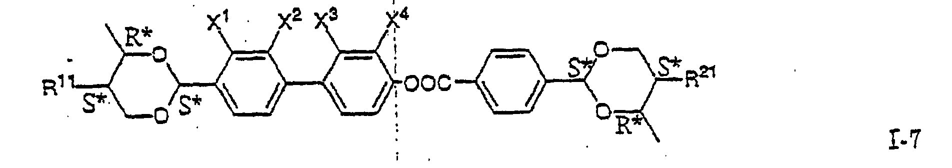 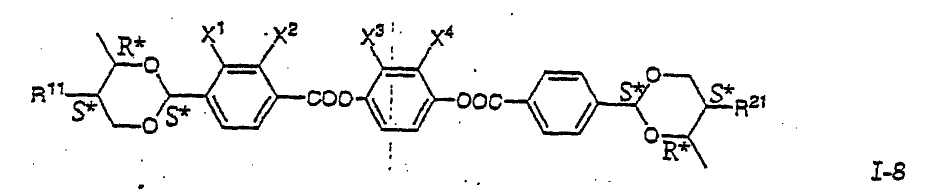 dans lesquelles
R¹¹ et R²¹ représentent indépendamment l'un de l'autre un alkyle ou alcényle ayant au plus 12 atomes de carbone, dans lequel un groupe méthylène est éventuellement remplacé par -O-, -COO- ou -OOC- ; et les substituants
X¹ à X⁴ représentent indépendamment les uns des autres un atome d'hydrogène ou de fluor.

6. Composés selon la revendication 4 ayant les formules 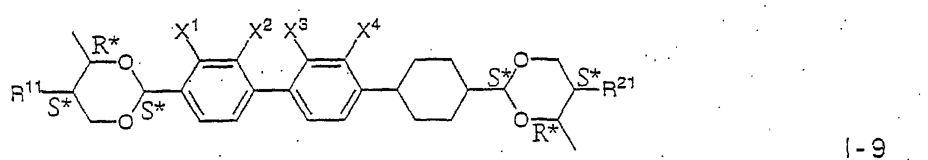 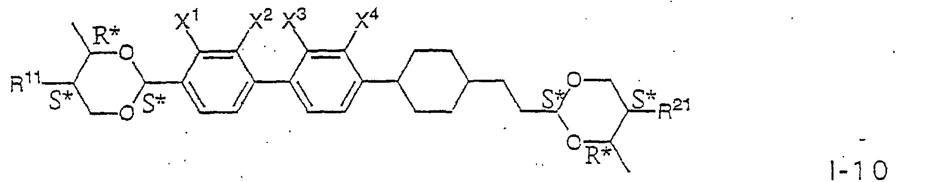 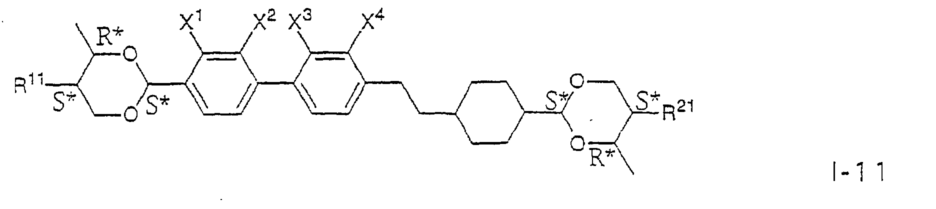 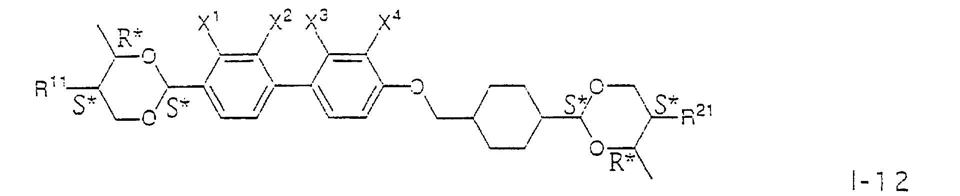 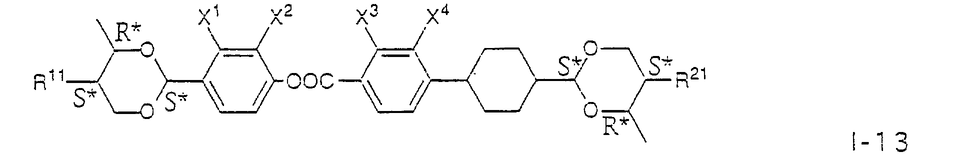 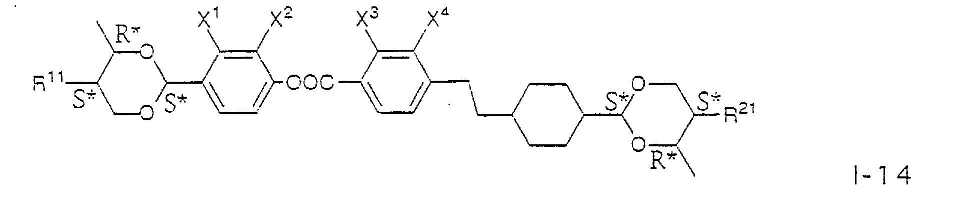 dans lesquelles
R¹¹ et R²¹ représentent indépendamment l'un de l'autre un alkyle ou alcényle ayant au plus 12 atomes de carbone, dans lequel un groupe méthylène est éventuellement remplacé par -O-, -COO- ou -OOC- ; et les substituants
X¹ à X⁴ représentent indépendamment les uns des autres un atome d'hydrogène ou de fluor.

7. Composés selon la revendication 4 ayant les formules 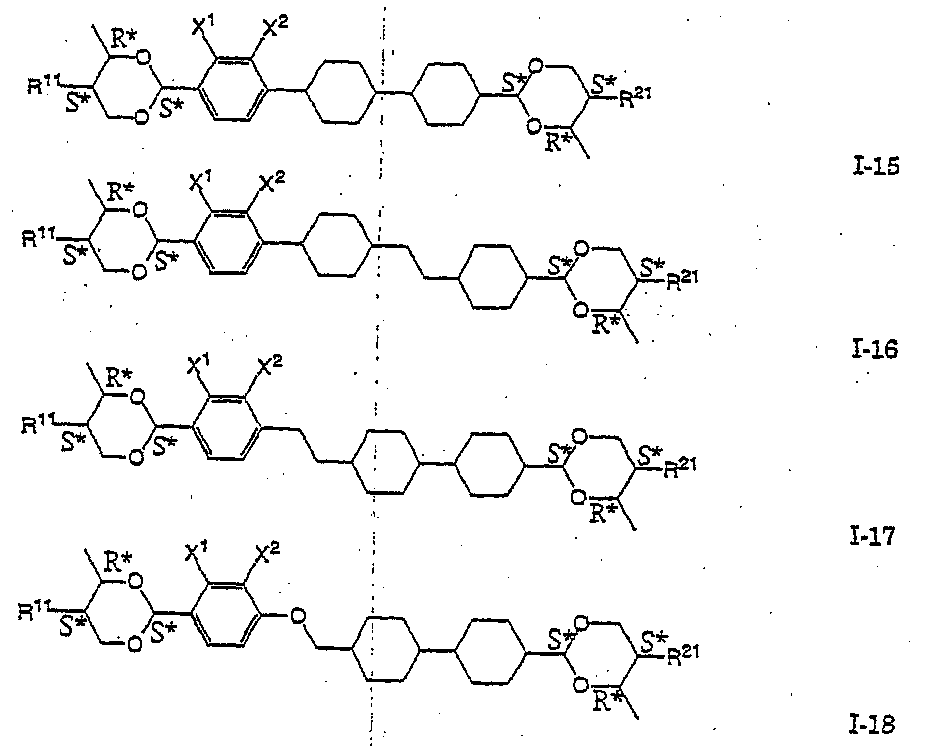 dans lesquelles
R¹¹ et R²¹ représentent indépendamment l'un de l'autre un alkyle ou alcényle ayant au plus 12 atomes de carbone, dans lequel un groupe méthylène est éventuellement remplacé par -O-, -COO- ou -OOC- ; et les substituants
X¹ à X⁴ représentent indépendamment les uns des autres un atome d'hydrogène ou de fluor.

8. Composés selon la revendication 4 ayant les formules 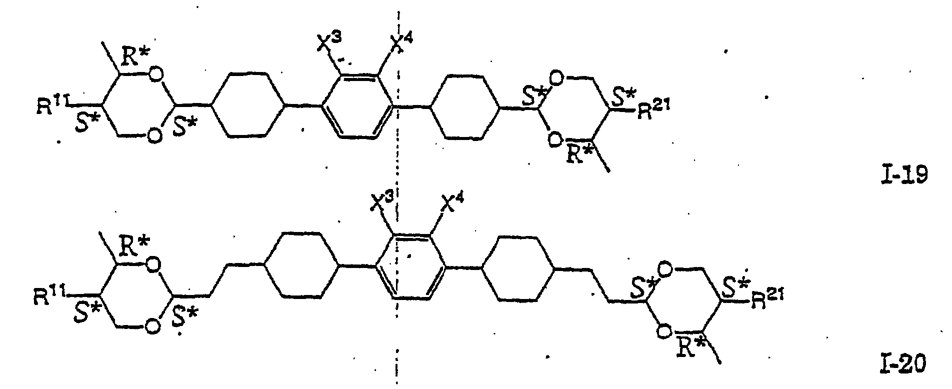 dans lesquelles
R¹¹ et R²¹ représentent indépendamment l'un de l'autre un alkyle ou alcényle ayant au plus 12 atomes de carbone, dans lequel un groupe méthylène est éventuellement remplacé par -O-, -COO- ou -OOC- ; et les substituants
X¹ à X⁴ représentent indépendamment les uns des autres un atome d'hydrogène ou de fluor.

9. Composés selon l'une des revendications 4 à 8, caractérisé en ce qu'au plus deux des groupes X¹ à X⁴ représentent le fluor.

10. Mélanges à cristaux liquides contenant au moins deux composants, caractérisés en ce qu'au moins un composant est un composé optiquement actif selon l'une des revendications 1 à 9.

11. Mélanges à cristaux liquides selon la revendication 10, caractérisés en ce que la proportion de composés optiquement actifs selon l'une des revendications 1 à 9 s'élève à 1-30% en poids.

12. Utilisation de composés selon l'une des revendications 1 à 9 pour des applications électrooptiques.

13. Utilisation de mélanges à cristaux liquides selon une des revendications 10 ou 11 pour des applications électrooptiques.

14. Dispositifs électrooptiques contenant des composés selon l'une des revendications 1 à 9.

15. Dispositifs électrooptiques contenant des composés selon l'une des revendications 10 ou 11.
